(19) Europäisches Patentamt / European Patent Office / Office européen des brevets

(11) **EP 3 911 942 B1**

(12) **FASCICULE DE BREVET EUROPEEN**

(45) Date de publication et mention
de la délivrance du brevet:
**06.09.2023 Bulletin 2023/36**

(21) Numéro de dépôt: **20701519.9**

(22) Date de dépôt: **15.01.2020**

(51) Classification Internationale des Brevets (IPC):
**G01N 21/65** (2006.01)     **G01J 3/44** (2006.01)
**G01N 21/15** (2006.01)

(52) Classification Coopérative des Brevets (CPC):
**G01N 21/65; G01J 3/44;** G01N 2021/157

(86) Numéro de dépôt international:
**PCT/IB2020/050302**

(87) Numéro de publication internationale:
**WO 2020/148671 (23.07.2020 Gazette 2020/30)**

(54) **SYSTÈME DE CAVITÉ OPTIQUE RÉSONANTE À RÉTROACTION OPTIQUE, ADAPTÉE À LA DÉTECTION DE TRACES DE GAZ PAR SPECTROMÉTRIE DE RAMAN**

OPTISCHES HOHLRAUMRESONATORSYSTEM MIT OPTISCHER RÜCKKOPPLUNG FÜR DEN NACHWEIS VON SPURENGASEN MITTELS RAMAN-SPEKTROMETRIE

OPTICAL CAVITY RESONATOR SYSTEM WITH OPTICAL FEEDBACK ADAPTED FOR TRACE GAS DETECTION BY RAMAN SPECTROMETRY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.01.2019 FR 1900454**

(43) Date de publication de la demande:
**24.11.2021 Bulletin 2021/47**

(73) Titulaire: **Ap2E**
**13857 Aix-En-Provence Cedex 3 (FR)**

(72) Inventeurs:
• **KATCHANOV, Alexandre**
**San Jose, California CA 95112 (US)**
• **JAULIN, Kevin**
**13770 Venelles (FR)**
• **STOLTMANN, Tim**
**13090 Aix-En-Provence (FR)**
• **CHOLAT, Pierre**
**13650 Meyrargues (FR)**
• **LONIGRO, Lucien**
**13290 Les Milles (FR)**
• **GILETTA, Bruno**
**13006 Marseille (FR)**

(74) Mandataire: **Marchand, André et al**
**OMNIPAT**
**24 Place des Martyrs de la Résistance**
**13100 Aix-en-Provence (FR)**

(56) Documents cités:
**WO-A1-03/031949**

• **MICHAEL HIPPLER: "Cavity-Enhanced Raman Spectroscopy of Natural Gas with Optical Feedback cw-Diode Lasers", ANALYTICAL CHEMISTRY, vol. 87, no. 15, 22 juillet 2015 (2015-07-22), pages 7803-7809, XP055640292, US ISSN: 0003-2700, DOI: 10.1021/acs.analchem.5b01462 cité dans la demande**

**Description**

La présente invention concerne l'analyse de gaz par spectrométrie de Raman. La spectrométrie de Raman se base sur l'effet Raman qui résulte d'une interaction inélastique d'un photon avec un milieu (molécule ou cristal). Cette interaction inélastique est appelée "diffusion Raman". L'existence d'une telle interaction inélastique implique que le milieu rencontré par le photon a absorbé ou fourni de l'énergie au photon. Ce transfert d'énergie se manifeste par un décalage en fréquence entre le photon incident et le photon diffusé à la suite de l'interaction. Lorsque le milieu absorbe de l'énergie du photon, la diffusion Raman est dite "avec décalage Stokes". Lorsque le milieu fournit de l'énergie au photon, la diffusion Raman est dite "avec décalage anti-Stokes". Il s'avère que ces décalages en fréquence sont caractéristiques de la composition chimique du milieu dans le cas d'un milieu (cristal, liquide ou gaz). La spectrométrie de Raman consiste donc à focaliser un faisceau de lumière monochromatique sur l'échantillon à étudier, à former un spectre de Raman comportant des raies spectrales ou raies Raman résultant de la diffusion Raman, et à déterminer les composants de l'échantillon et leurs concentrations en comparant les raies spectrales de Raman du spectre à des données théoriques et expérimentales. En effet, l'intensité des raies spectrales de Raman est proportionnelle au nombre de molécules rencontrées par le faisceau incident et aux propriétés relatives à la diffusion Raman des molécules rencontrées, ainsi que l'intensité du faisceau d'excitation. Il en résulte que la spectrométrie de Raman nécessite un faisceau laser monochromatique pour obtenir une résolution spectrale suffisante, et de puissance suffisante, sachant que l'intensité des raies de Raman est bien plus faible que celui du faisceau incident. En pratique, la puissance de la source laser requise atteint plusieurs centaines de milli Watts, voire plusieurs dizaines de Watts, ce qui nécessite l'utilisation de lasers puissants et donc coûteux et encombrants.

La spectrométrie de Raman s'est largement développée depuis l'apparition du laser, en raison des avantages qu'elle procure, notamment sa capacité à déterminer la nature d'un très grand nombre de différentes molécules, présentes dans le milieu analysé, sans avoir à changer de source laser, contrairement à la spectrométrie par absorption. Peu après l'apparition des premiers spectromètres de Raman, on a donc proposé de placer le milieu à analyser dans une cavité optique résonnante dans laquelle la puissance d'un faisceau laser peut être accumulée ("power buildup").

La publication [1] décrit un spectromètre de Raman pour la détection de gaz, dans lequel un échantillon est placé dans une cavité optique excitée par un laser Ar-ion fournissant une puissance de 0,5 W, la cavité optique produisant une puissance d'excitation de l'échantillon de 160 W. Ce système présente une limite de détection d'une espèce chimique de l'ordre de quelques dizaines de ppm (parties par million). Cependant, les lasers Ar-ion restent trop encombrants pour de nombreuses applications nécessitant l'usage d'analyseurs de gaz très compacts.

Le développement de spectromètres de Raman a donc plus récemment fait l'objet d'intenses recherches pour tenter de les rendre plus compacts. Des progrès récents dans le domaine du verrouillage par rétroaction optique de diodes laser de faible puissance sur des cavités optiques de grande finesse, ont permis de générer des faisceaux laser très intenses dans ces cavités. A titre d'exemple, la publication [2], décrit une cavité optique résonnante excitée par une diode laser. Une petite fraction faisceau laser produit par la diode laser est introduite dans la cavité optique par l'un des miroirs de la cavité, et subit de multiples réflexions entre les miroirs de la cavité, ce qui produit une accumulation de puissance. La largeur de la bande spectrale du faisceau produit par la diode laser est initialement plus grande de plusieurs ordres de grandeur que la largeur de mode de la cavité. Dans cette situation, l'efficacité du couplage du faisceau produit par une diode laser, typiquement d'une largeur spectrale de 1 à 10 MHz, sur un mode de résonance de la cavité, d'une largeur spectrale de 10 kHz, peut être inférieure à une fraction de pourcent. L'injection directe d'une diode laser dans une cavité en vue d'accumuler de la puissance s'avère donc très inefficace. Cependant, dans cette publication, une petite fraction du faisceau résonnant circulant dans la cavité, et donc ayant la largeur spectrale du mode de résonance de la cavité, est extraite de la cavité par l'un des miroirs de cavité et renvoyée vers la diode laser où elle subit une amplification d'un facteur supérieur à un millier par émission stimulée. Ce faisceau à bande spectrale étroite est alors réinjecté dans la cavité, avec une efficacité bien plus grande, et ainsi de suite. Après plusieurs cycles, la largeur spectrale du faisceau émis par la diode laser et se réduit à la bande spectrale beaucoup plus étroite d'un mode de résonnance de la cavité optique. Dans l'exemple précédent, la largeur spectrale du faisceau laser émis passe d'environ 10 Mhz à quelques Hertz. L'énergie émise par la diode laser peut être ainsi transmise à la cavité avec un taux de transmission élevé, dès lors que la fréquence du faisceau laser se trouve suffisamment proche de celle d'un mode de résonnance de la cavité. Le faisceau émis par la diode laser peut alors se verrouiller en fréquence sur ce mode de résonnance de la cavité optique, et rester ainsi verrouillé jusqu'à ce que la fréquence intrinsèque de la diode laser sorte d'une bande de fréquence de verrouillage incluant la fréquence du mode de résonnance de la cavité. Cet effet de réduction de la largeur spectrale et de verrouillage est appelé "verrouillage par rétroaction optique". La largeur de la bande de fréquence de verrouillage dépend de la fraction de la puissance lumineuse susceptible d'être extraite de la cavité en tant que faisceau de rétroaction optique. Typiquement, avec un coefficient de rétroaction de seulement -30 dB ou moins, la bande de fréquence de verrouillage peut être aussi large que l'intervalle de fréquences entre deux modes de résonnance longitudinaux adjacents de la cavité, cet intervalle étant appelé "plage spectrale libre" FSR ("free spectral range") de la cavité optique.

Pour obtenir un verrouillage de la rétroaction optique, il est nécessaire d'accorder la phase du faisceau de rétroaction à la phase du faisceau circulant dans la cavité optique. Il est également nécessaire que la fréquence optique intrinsèque de la diode laser traverse la bande de verrouillage d'un mode de la cavité. Plusieurs solutions plus moins complexes ont été proposées pour maintenir ces accords de phase et de fréquence (voir par exemple la publication [3]). Cependant, lorsque les accords de phase et de fréquence sont perdus, il est difficile de les restaurer automatiquement. En réalité, ces pertes d'accord se produisent fréquemment, en particulier dans des environnements industriels subissant des perturbations mécaniques, thermiques et électriques, de sorte qu'il est difficile d'obtenir des mesures.

Le brevet US5432610, ainsi que les brevets dérivés US5642375, US5684623, US5835522 et US6222860, décrivent des spectromètres de Raman utilisant une diode laser couplée à une cavité optique. Cependant, il semble que ces systèmes rencontrent des problèmes de pertes fréquentes de la rétroaction optique, ce qui conduit à une perte intermittente du signal optique. D'une manière générale, ces brevets n'indiquent pas comment il serait possible de générer un signal de Raman qui soit stable et reproductible, pour pouvoir obtenir des mesures fiables et précises.

Plus récemment, les publications [4] et [5] décrivent un spectromètre de Raman comprenant une cavité linéaire associée à une architecture complexe faisant intervenir de nombreux composants. Le maintien de l'excitation de la cavité optique dans le même mode est réalisé en appliquant à la tension d'alimentation de la diode laser un signal en dent de scie de faible amplitude de manière à ce que la fréquence du faisceau laser produit par la diode balaye une certaine plage de fréquences autour de la fréquence d'un unique mode de résonnance de la cavité optique. De cette manière, le faisceau laser est toujours verrouillé en fréquence sur cet unique mode de résonnance, et reste monochromatique. Ainsi, pour s'assurer que toujours le même mode de résonnance est activé, il suffit de vérifier que le pic de transmission de la cavité correspondant à ce mode de résonnance, reste au milieu de chaque rampe montante et descendante du courant en dent de scie, et d'ajuster le courant d'alimentation de la diode pour maintenir cette condition. Cette méthode fournit également un moyen d'ajuster la phase de la rétroaction optique, en observant la symétrie du pic de transmission.

Ce procédé semble favorable pour produire une excitation persistante et répétitive de la cavité optique dans un mode de résonnance. Cependant, cette méthode reste sensible aux perturbations mécaniques : une vibration peut entrainer une perte de l'accord de phase, ce qui interrompt la résonnance de la cavité, jusqu'à ce que la phase de la rétroaction optique se rapproche suffisamment de sa valeur optimale. Pendant ce temps, il n'est bien sûr pas possible d'obtenir des mesures. Un autre problème résulte de l'utilisation d'un signal en dent de scie ayant des rampes montantes et descendantes de durées égales, En effet, un tel signal empêche d'obtenir un rapport cyclique (rapport entre le temps pendant lequel se produit le verrouillage dans le mode de résonnance de la cavité optique, et le temps écoulé) supérieur à 50%. En effet, le verrouillage de la diode laser sur un mode de résonnance de la cavité se produit non seulement pendant la rampe montante du courant d'alimentation de la diode, mais également pendant la rampe descendante, plus courte. En pratique, les propriétés de la rétroaction optique ne permettent pas d'obtenir un pic de transmission de durée maximum dans la cavité optique durant chacune des deux rampes montante et descendante, tout en maintenant la symétrie des pics de transmission et sans changer l'ajustement de la phase. Ainsi, il est nécessaire de prévoir une marge dans l'amplitude de variation du courant d'alimentation. Cette marge conduit à limiter le rapport cyclique à environ 50% ou moins, et donc à limiter l'énergie utilisable, fournie par la diode laser.

Il est donc souhaitable de réaliser un système d'analyse de gaz par spectrométrie de Raman qui soit compact, stable, robuste, et capable de fournir des mesures de grande sensibilité. Il est également souhaitable d'utiliser plus efficacement l'énergie produite par la source laser. Les demandes de brevet WO 03/031949 et WO 2018/060285 décrivent des systèmes de cavité optique utilisés dans le domaine de la spectrométrie par absorption. Dans ces systèmes, une cavité optique est excitée par une diode laser calée par rétroaction optique, séquentiellement sur plusieurs modes de résonnance de la cavité optique. Ces systèmes cherchent à minimiser la puissance optique dans la cavité, afin d'éviter la saturation des raies d'absorption. Cependant, ces systèmes présentent l'avantage d'offrir une très grande robustesse de fonctionnement, et une restauration rapide de la puissance optique dans la cavité en cas d'importante perturbation. Cependant, ces systèmes ne semblent pas convenir pour la spectrométrie de Raman car ils se basent sur l'excitation séquentielle de plusieurs modes de la cavité. Il en résulte que la lumière accumulée dans la cavité n'est pas globalement monochromatique, mais présente une fréquence qui varie périodiquement.

La présente invention propose d'adapter les dispositions de ces systèmes de cavité optique aux contraintes de la spectrométrie de Raman, notamment en limitant la plage de balayage des modes de la cavité optique à une valeur inférieure à la résolution spectrale du spectromètre de Raman, et en augmentant autant que possible la puissance optique accumulée dans la cavité optique.

Des modes de réalisation concernent un procédé d'analyse de gaz par spectrométrie de Raman, le procédé comprenant des étapes consistant à : appliquer un signal de commande à une source laser afin qu'elle produise un faisceau laser balayant une plage de fréquences incluant des fréquences de plusieurs modes de résonnance d'une cavité optique incluant des gaz à analyser, la largeur de la plage de fréquence étant inférieure à la résolution d'un spectromètre de Raman, fournir le faisceau laser à une entrée de la cavité optique, extraire de la cavité optique un faisceau de rétroaction, ajuster une phase et une amplitude du faisceau de rétroaction, envoyer le faisceau de rétroaction ajusté en phase et en amplitude, sur la source laser, durant le balayage de la plage de fréquences, mesurer des variations d'intensité lumineuse du faisceau laser dans la cavité optique, et détecter des pics d'intensité lumineuse dans les variations d'intensité lumineuse, chaque pic d'intensité lumineuse correspondant à un mode de résonnance de la cavité optique, la phase du faisceau de rétroaction étant ajustée pour réduire un écart entre un instant médian et un instant d'apparition d'un sommet, d'un des pics d'intensité lumineuse, l'amplitude du faisceau de rétroaction étant ajustée pour réduire au moins un intervalle d'intensité nulle entre les pics d'intensité lumineuse, et acquérir à l'aide d'un capteur du spectromètre de Raman, des mesures de spectre de lumière diffusée inélastiquement sous l'effet du faisceau laser présent dans la cavité optique, pour déterminer la nature et/ou la concentration des gaz présents dans la cavité optique.

Selon un mode de réalisation, le signal de commande de la source laser présente une forme périodique, et le capteur de spectromètre comprenant une pluralité de cellules photosensibles, reçoit une partie de la lumière diffusée inélastiquement et est commandé de manière à enchaîner des phases d'exposition en alternance avec des phases de lecture durant lesquelles les mesures de spectre sont fournies par les cellules photosensibles, le procédé comprenant, durant chaque phase d'exposition, des étapes consistant à : obtenir une valeur intégrale de mesures d'intensité lumineuse du faisceau laser dans la cavité optique, et/ou d'intensité lumineuse d'une partie de la lumière diffusée élastiquement sous l'effet du faisceau laser présent dans la cavité optique, et normaliser chacune des mesures de spectre obtenues à l'issue de la phase d'exposition en fonction de la valeur intégrale, pour obtenir un spectre normalisé. Selon un mode de réalisation, les phases d'exposition présentent une durée égale à un multiple de la période du signal de commande de la source laser, le début de chaque phase d'exposition étant synchronisé avec le signal de commande de la source laser.

Selon un mode de réalisation, chaque phase d'exposition est démarrée et interrompue durant des périodes où aucune lumière n'est présente dans la cavité optique.

Selon un mode de réalisation, le procédé comprend des étapes consistant à : couper périodiquement l'alimentation de la source laser, pour mesurer un courant de noir dans un photodétecteur fournissant les mesures de variation d'intensité lumineuse du faisceau laser dans la cavité optique, et corriger les mesures de variations d'intensité lumineuse du faisceau laser dans la cavité optique, à l'aide d'une moyenne de mesures de courant de noir.

Selon un mode de réalisation, les coupures de l'alimentation de la source laser sont effectuées en dehors des phases d'exposition.

Selon un mode de réalisation, le procédé comprend des étapes consistant à : couper l'alimentation de la source laser durant une phase d'arrêt s'étendant sur au moins une phase d'exposition consécutive, et pour chaque cellule photosensible du capteur de spectromètre : mesurer un courant de noir durant la phase d'arrêt, corriger un courant de noir moyen mémorisé pour la cellule photosensible en fonction de la mesure de courant de noir obtenue pour la cellule photosensible, et corriger chaque mesure de spectre fournie par la cellule photosensible en fonction du courant de noir moyen mémorisé pour la cellule photosensible, la mesure de courant de noir pouvant être précédée de plusieurs mesures non prises en compte pour effacer les cellules photosensibles.

Selon un mode de réalisation, le faisceau laser fourni en entrée de la cavité optique et le faisceau de rétroaction sont transmis par un même chemin optique entre la source laser et l'entrée de la cavité optique.

Selon un mode de réalisation, l'amplitude du faisceau de rétroaction est ajustée par un module d'isolation de Faraday ajustable installé sur le chemin optique entre la source laser et l'entrée de la cavité optique, le module d'isolation de Faraday atténuant l'amplitude du faisceau de rétroaction et laissant passer le faisceau laser issu de la source laser.

Selon un mode de réalisation, le module d'isolation de Faraday est asservi en fonction des mesures d'intensité lumineuse du faisceau laser dans la cavité optique, pour minimiser les intervalles d'intensité nulle entre les pics d'intensité lumineuse, l'asservissement du module d'isolation de Faraday pouvant être réalisé en agissant sur sa température.

Des modes de réalisation peuvent également concerner un dispositif d'analyse de gaz par spectrométrie de Raman, comprenant une source laser, une cavité optique pour recevoir des gaz à analyser et comportant une entrée par laquelle est introduit un faisceau laser émis par la source laser, un organe de commande produisant un signal de commande de la source laser, un composant pour extraire un faisceau de rétroaction de la cavité optique, des composants pour ajuster le faisceau de rétroaction en phase et en amplitude et pour envoyer le faisceau de rétroaction ajusté en phase et en amplitude sur la source laser, un organe de mesure de variations d'intensité lumineuse du faisceau laser dans la cavité, et un spectromètre de Raman pour acquérir des mesures de spectre de lumière diffusée sous l'effet du faisceau laser présent dans la cavité optique, le dispositif d'analyse étant configuré pour mettre en oeuvre le procédé défini précédemment.

Selon un mode de réalisation, la source laser est configurée pour balayer la plage de fréquences sans saut de mode.

Selon un mode de réalisation, la cavité optique est en V et comprend un miroir de couplage formant l'entrée de la cavité optique, et deux miroirs de fond de cavité, les miroirs de fond de cavité présentant une réflectivité plus grande ou identique à celle du miroir de couplage. Selon un mode de réalisation, les miroirs formant la cavité optique présentent des rayons de courbure adaptés pour ajuster la forme du faisceau à l'intérieur de la cavité optique en une zone de collecte de la lumière diffusée dans la cavité sous l'effet du faisceau laser.

Selon un mode de réalisation, la lumière diffusée dans la cavité optique est observée au travers de l'un des miroirs de fond de cavité, les miroirs de fond de cavité présentant un revêtement ayant un coefficient de réflexion maximum pour le faisceau réfléchi dans chacun des modes de résonnance de la cavité optique, et ayant un coefficient de transmission maximum pour au moins une raie de Stockes des raies de Raman.

Selon un mode de réalisation, la source laser est de l'un des types suivants : une diode laser à rétroaction répartie, une diode laser à réflecteur de Bragg distribué, une diode de type Fabry-Perot stabilisée par un réseau de diffraction de Bragg en volume ou holographique, ou par un filtre à bande étroite, un laser associé à un dispositif d'ajustement de la fréquence d'émission, un laser à émission de surface et à cavité verticale, couplé à un miroir à membrane diélectrique.

Selon un mode de réalisation, le dispositif comprend un actionneur couplé à un miroir pour modifier une longueur de chemin optique entre la source laser et l'entrée de la cavité optique, afin d'ajuster la phase du faisceau de rétroaction.

Des exemples de réalisation de l'invention seront décrits dans ce qui suit, à titre non limitatif en relation avec les figures jointes parmi lesquelles :

La figure 1 représente schématiquement un système d'analyse de gaz par spectrométrie de Raman, selon un mode de réalisation,
Les figures 2 à 5 représentent des chronogrammes de signaux observés dans le système d'analyse de gaz de la figure 1.

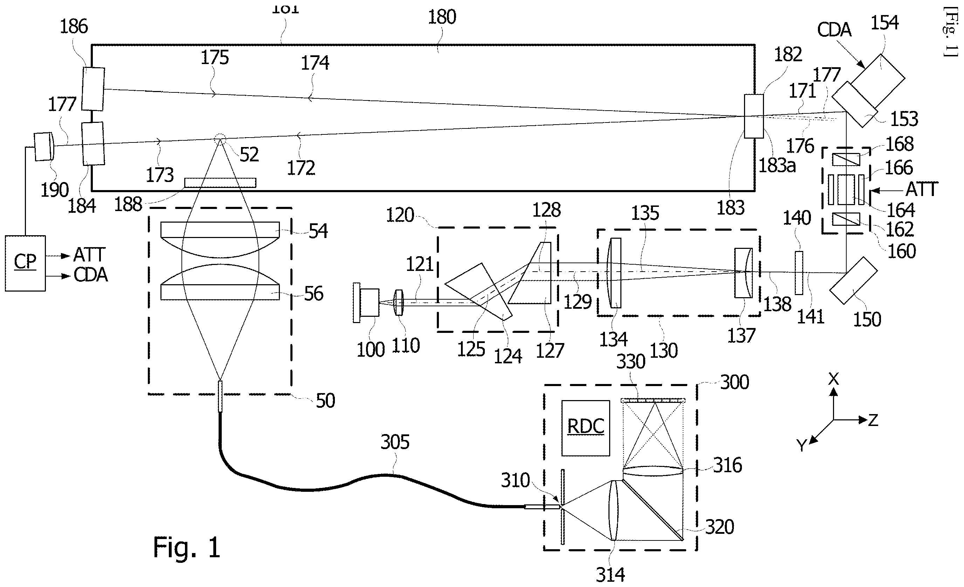

La figure 1 représente un système d'analyse de gaz par spectrométrie de Raman, selon un mode de réalisation. Le système d'analyse de gaz comprend une source laser 100, un module optique 120 de mise en forme du faisceau laser émis par la source laser, un module optique 130 d'accord de mode de résonnance, un module optique 160 d'atténuation de rétroaction optique, une cavité optique 180, un photodétecteur 190, et un dispositif optique 50 pour recueillir une partie de la lumière diffusée par les gaz présents dans la cavité optique 180 sous l'effet du faisceau laser dans la cavité optique, le dispositif optique 50 étant couplé à une entrée 310 d'un spectromètre de Raman 300.

La cavité optique 180, de forme en V, à deux branches, comprend un corps de cavité 181 délimitant la cavité 180, un miroir d'entrée ou de couplage 182 et deux miroirs de fond de cavité 184, 186, ces trois miroirs étant fixés sur le corps de cavité 181. Le corps de cavité 181 est configuré pour permettre à des gaz à analyser d'y circuler, et à la lumière de se propager entre les miroirs 182, 184, 186. Le corps de cavité 181 peut être réalisé par exemple en acier inoxydable.

La source laser 100 est configurée pour émettre un faisceau lumineux dans un unique mode longitudinal, dans le domaine des ultraviolets, dans le domaine visible du proche infrarouge. La source laser 100 présente également la capacité de modifier la fréquence du faisceau laser émis, par exemple sans saut de mode, en réponse à des variations d'un de ses paramètres de contrôle tels que, par exemple, son courant d'alimentation ou sa température, ou encore d'autres paramètres dépendant de la structure de la source laser. En outre, la plage de variation de la fréquence d'émission de la source laser 100, s'étend sur plus de la largeur de plusieurs plages spectrales libres FSR de la cavité 180. Le faisceau issu de la source laser 100 est émis dans une direction Z. La figure 1 est située dans un plan XZ, la direction Y étant perpendiculaire au plan XZ.

La source laser 100 peut être par exemple une diode laser de type à rétroaction répartie DFB (Distributed FeedBack). Il est possible d'utiliser d'autres sources laser, comme par exemple une diode laser de type réflecteur de Bragg distribué DBR (Distributed Bragg reflector). La source laser peut également être une diode de type Fabry-Perot stabilisée par un élément externe de sélection de fréquence, tel qu'un réseau de diffraction de Bragg en volume VBG (Volume Bragg Grating), un réseau de diffraction, ou un filtre à bande étroite. La source laser peut être également un laser associé à un dispositif complexe d'ajustement de la fréquence d'émission, ou un laser à émission de surface et à cavité verticale VCSEL (Vertical Cavity Surface Emitting Laser) couplé à un miroir à membrane diélectrique, dont la fréquence d'émission peut être ajustée par une tension appliquée entre le substrat laser et le miroir.

Le faisceau émis par la source laser 100 traverse un module optique 110 de collimation permettant de réduire la divergence du faisceau émis, à la fois dans deux directions perpendiculaires à la direction de propagation du faisceau. La collimation du faisceau laser émis est classiquement mise en oeuvre en spectrométrie utilisant une diode laser, pour faciliter la fourniture du faisceau laser à une cellule d'absorption et à un photodétecteur en sortie de la cellule. En spectrométrie de Raman, basée sur l'utilisation d'une cavité optique, la conformation du faisceau laser doit être réalisée d'une manière plus précise. En effet, pour obtenir un transfert d'énergie lumineuse efficace, le faisceau laser doit être accordé sur le mode de résonnance fondamental de la cavité, ce qui requiert d'ajuster les distributions d'amplitude et de phase du faisceau laser pour les rendre aussi proches que possible de celles du mode de résonnance fondamental de la cavité. Dans le mode de réalisation représenté sur la figure 1, l'accord de mode est réalisé par les modules optiques 110, 120 et 130.

Le module 110 peut être une simple lentille qui peut être asphérique, ou une lentille doublement acylindrique. Le module 110 peut également être une combinaison de plusieurs lentilles plus ou moins complexes ou d'autres composants optiques.

De nombreuses diodes laser produisent des faisceaux ayant une forme elliptique présentant une excentricité élevée et/ou un astigmatisme important. Le faisceau laser collimaté 121 en sortie du module 110 peut donc avoir des largeurs et des divergences très différentes selon la direction considérée. Le faisceau laser collimaté 121 en sortie du module 110 est donc ensuite traité par le module optique 120 de mise en forme configuré pour produire un faisceau ayant une forme sensiblement circulaire. Selon un mode de réalisation, le module optique 120 de mise en forme comprend une paire de prismes anamorphiques 124, 127, permettant d'élargir le faisceau 121 dans une direction. D'après l'orientation des prismes 124, 125 indiquée sur la figure 1, le faisceau 121 en sortie du module optique 110 présente une section elliptique (dans le plan XY) dont le plus grand axe est orienté suivant la direction Y. Les deux prismes 124, 127 élargissent le faisceau dans la direction X afin de rendre les largeurs du faisceau dans les directions X et Y aussi proches l'une de l'autre que possible. A titre d'exemple, l'excentricité du faisceau laser produite par la source laser est de 2,8. Cependant, les diodes laser dans le domaine visible peuvent fournir un faisceau laser présentant une excentricité de 4.

Selon un autre mode de réalisation, le module optique 120 peut être réalisé à l'aide de lentilles cylindriques.

Le faisceau laser 129 mis en forme, en sortie du module optique 120 est ensuite traité par le module optique 130 d'accord de mode de résonnance. Le module 130 est configuré pour ajuster les paramètres spatiaux du faisceau 129 afin qu'ils soient proches des paramètres spatiaux du faisceau du mode fondamental de la cavité optique 180 (accord de mode). Cette condition d'accord de mode peut être obtenue lorsque les distributions en amplitude et en phase dans les directions X et Y du faisceau 171 au niveau miroir d'entrée 182, coïncident ou sont très proches de celles du faisceau 172 correspondant au mode fondamental de la cavité. Le module optique 130 peut comprendre une ou plusieurs lentilles ou une combinaison de différents composants optiques. Dans l'exemple de la figure 1, le module optique 130 comprend deux lentilles sphériques 134, 137.

Le faisceau 138 en sortie du module optique 130 est dirigé vers le miroir d'entrée 182 de la cavité optique 180 par deux miroirs 150, 153, afin que le faisceau 171 réfléchi par le miroir 153 soit colinéaire avec l'image du faisceau 172 du mode fondamental circulant dans la cavité 180. Le miroir 153 est monté sur un actionneur 154 tel qu'un transducteur piézo-électrique, permettant d'ajuster la longueur du chemin optique entre la sortie de la source laser 100 et le miroir de couplage 182 à l'entrée de la cavité optique 180, et ainsi d'ajuster la phase du faisceau de rétroaction issu de la cavité 180 et provenant du miroir de fond de cavité 184. Ainsi, les conditions du verrouillage de la rétroaction optique peuvent être satisfaites.

Entre les miroirs 150 et 153 se trouve le module optique 160 d'atténuation ajustable de l'intensité de la rétroaction optique. La fonction du module 160 est d'ajuster à une valeur optimale le rapport d'intensité entre le faisceau de rétroaction et le faisceau incident issu de la source laser 100, dans la mesure où la largeur de la bande de verrouillage de la rétroaction est proche de celle de la plage spectrale libre FSR sans être supérieure à celle-ci. La valeur optimale de l'intensité du faisceau de rétroaction dépend des caractéristiques des miroirs 182, 184, 186 formant la cavité optique 180, des caractéristiques de la source laser 100 et de l'efficacité du couplage entre la source laser et la cavité optique. Typiquement, le rapport d'atténuation nécessaire pour atteindre cette valeur optimale est situé entre 25 et 35 dB. Il s'avère que la valeur optimale de l'intensité du faisceau de rétroaction est sensible à d'infimes variations, au point qu'elle varie au sein d'un même lot de fabrication. Cette valeur optimale varie également dans le temps, notamment en raison du vieillissement des composants du système d'analyse de gaz, et des pertes intra cavité dues notamment à la présence de certains gaz.

En théorie, n'importe quel atténuateur ayant un coefficient de transmission ajustable peut être utilisé. Ainsi, le module d'atténuation 160 peut être par exemple un filtre de densité neutre, ou même une paire de polariseurs ayant des polarisations presque opposées (nearly crossed polarisers). Cependant, de tels polariseurs peuvent entrainer une perte significative de la puissance du faisceau injecté dans la cavité, ce qui n'est pas souhaitable dans le cadre d'un système de spectrométrie de Raman. En pratique, il est nécessaire, non seulement de pouvoir ajuster la plage de verrouillage de la rétroaction optique, mais également de pouvoir injecter dans la cavité le plus possible de puissance lumineuse, ce qui n'est pas le cas dans les systèmes de l'art antérieur (par exemple celui décrit dans la demande de brevet WO 03/031949).

Le faisceau 172 issu de la transmission du faisceau 171 par le miroir de couplage 182, est réfléchi par un premier miroir 184 des miroirs de fond de cavité. Le faisceau réfléchi 173 par le miroir 184 se réfléchit sur le miroir de couplage 182. Le faisceau 174 réfléchi par le miroir de couplage 182 est renvoyé vers un second miroir 186 des miroirs de fond de cavité, qui renvoie un faisceau 175 en direction du miroir de couplage 182. La partie du faisceau de cavité 173 provenant du miroir 184 et traversant le miroir de couplage 182 constitue le faisceau de rétroaction. La partie 176 du faisceau 175 provenant du miroir 186 et traversant le miroir de couplage 182 présente la même direction que la partie 177 du faisceau incident 171, issu de la source laser 100 et qui est réfléchi directement par le miroir de couplage 182 sans entrer dans la cavité.

Le faisceau 172 pénétrant dans la cavité optique 180 excite l'un des systèmes de mode de cavité. L'un des systèmes de mode de cavité est polarisé dans le plan d'incidence (XZ) du miroir de couplage 182, et un autre de ces systèmes de mode de cavité est polarisé perpendiculairement au plan d'incidence YZ. Il en résulte que pour exciter l'un de ces systèmes de mode de cavité, l'axe de transmission du polariseur de sortie 168 du module d'atténuation 160, est orienté parallèlement à l'axe X ou Y. L'axe de transmission du polariseur d'entrée 162 est donc orienté approximativement à 45° par rapport au plan XZ. Cela ne coïncide pas avec la polarisation du faisceau incident 138 provenant du module 130. La polarisation du faisceau 138 est donc tournée d'un angle d'environ 45°, par exemple au moyen d'une lame demi-onde 140 disposée entre les modules 130 et 160. Le faisceau en sortie de la lame 140 présente ainsi une polarisation correspondant à l'orientation du polariseur d'entrée 162.

Le photodétecteur 190 permet d'observer le faisceau à l'intérieur de la cavité optique 180, au travers du miroir 184 de fond de cavité. Le photodétecteur 190 est par exemple constitué d'une photodiode. Les signaux issus du photodétecteur 190 sont traités et analysés par un processeur CP configuré pour générer des signaux de commande ATT et CDA permettant de contrôler l'actionneur 154 et le module d'atténuation 160

Le faisceau laser circulant dans la cavité optique, incluant les parties 172 à 175, est utilisé comme source pour produire une diffusion de Raman. La lumière ainsi diffusée est collectée par le module optique 50 comprenant deux lentilles 54, 56 de grande ouverture numérique, qui transmettent, en dehors du corps de cavité 181, une image de la lumière diffusée en une zone 52 de recueil de la lumière diffusée dans la cavité. Le module optique 50 est couplé à l'entrée d'un spectromètre de Raman, directement ou par l'intermédiaire d'une liaison optique 305. La liaison optique 305 peut être une fibre optique ayant un diamètre approprié ou un faisceau de fibres optiques.

Le spectromètre de Raman peut comprendre classiquement une fente d'entrée 310 couplée à une lentille de collimation 314 ayant une grande ouverture numérique, un réseau de diffraction 320, une lentille 316, et un capteur de spectromètre 330 permettant de constituer un spectre à partir de la lumière issue du réseau de diffraction 320. Le faisceau lumineux pénétrant par la fente 310 est transmis au réseau de diffraction 320 par la lentille 314. Le faisceau lumineux issu du réseau de diffraction 320 est transmis au capteur 330 par la lentille 316. Le capteur 330 comprend une barrette de plusieurs centaines de cellules photosensibles qui reçoivent la lumière transmise par la lentille 316 au travers d'un filtre qui répartit les longueurs d'onde reçues sur les cellules photosensibles du capteur 330, de manière à ce que chaque cellule du capteur ne reçoive qu'une plage de longueurs d'onde réduite appartenant à la bande de longueurs d'onde correspondant au spectre à générer. Le filtre peut être par exemple de type Fabry-Perot, ou de type linéaire variable. Grâce à un tel filtre, les longueurs d'onde transmises aux cellules photosensibles du capteur 330 peuvent exclure la bande de longueur d'onde du faisceau laser dans la cavité optique, de manière à ce que les cellules photosensibles ne reçoivent que la lumière diffusée inélastiquement dans la cavité, c'est-à-dire le spectre de Raman.

Le spectre généré par le capteur 330 peut être analysé à l'aide de techniques d'analyses spectrales connues en spectrométrie de Raman, pour déterminer la nature et/ou la concentration des gaz présents dans la cavité optique. Ces techniques d'analyse spectrale peuvent consister à détecter et mesurer dans le spectre généré, des fréquences et des amplitudes de raies de Raman à partir desquelles peuvent être déduites la nature et éventuellement la concentration des gaz présents dans la cavité optique.

Le miroir de couplage 182 comprend sur sa face tournée vers l'intérieur de la cavité 180 un revêtement diélectrique 183 présentant un coefficient de réflexion R élevé (proche de 1). La face du miroir 182 tournée vers l'extérieur de la cavité peut être couverte d'un revêtement antireflets 183a. Typiquement, la quantité 1-R est choisie dans la gamme allant de quelques ppm à plusieurs centaines de ppm. Dans certaines applications, la quantité 1-R peut même atteindre quelques pourcents.

Selon un mode de réalisation, les miroirs de fond de cavité 184, 186 sont identiques au miroir 182. Il en résulte qu'en un pic d'intensité lumineuse observé dans la cavité, le faisceau laser circulant dans la cavité sort de celle-ci dans quatre directions, à chaque fois qu'il rencontre l'un des miroirs 182, 184, 186.

Selon un autre mode de réalisation, les miroirs de fond de cavité 184, 186 présentent une réflectivité beaucoup plus grande que celle du miroir 182. Cette disposition permet de réduire les pertes d'énergie dues à la transmission du faisceau laser circulant dans la cavité par les miroirs de fond de cavité 184, 186, et également d'augmenter l'accumulation de puissance lumineuse dans la cavité optique 180, par un facteur pouvant atteindre 4, et une augmentation en conséquence de l'intensité du signal de Raman.

Le gain de puissance lumineuse accumulée dans la cavité peut atteindre quatre si l'on supprime les pertes significatives de puissance lumineuse dues à l'absorption des gaz remplissant la cavité 180 et lorsque les miroirs 184, 186 de fond de cavité présentent des coefficients de réflexion très élevés, bien supérieurs à leurs coefficients de transmission. Dans ce cas seulement, une faible part de la puissance lumineuse sort de la cavité par les miroirs 184, 186, et l'accumulation de puissance lumineuse dans la cavité est telle que le faisceau 176 sortant de la cavité par le miroir de couplage 182, et le faisceau 177 réfléchi directement par ce dernier, sont colinéaires et présentent des puissances sensiblement identiques. Comme les faisceaux 176 et 177 présentent des phases opposées, la puissance combinée de ceux-ci est voisine de zéro. Ainsi, toute la puissance transmise par la source laser à la cavité sort de celle-ci dans une seule direction au lieu de quatre, les trois autres faisceaux sortant de la cavité présentant une intensité négligeable. Ces dispositions permettant d'injecter dans la cavité optique toute l'énergie disponible fournie par la source laser, tout en évitant les pertes, est appelée "accord d'impédance" dans la théorie des cavités optiques résonnantes.

Pour obtenir des signaux de Raman exploitables, il peut être avantageux d'améliorer le couplage entre la lumière diffusée dans la cavité optique 180 et la fente d'entrée 310 du spectromètre de Raman. La fente d'entrée d'un spectromètre de Raman classique présente une largeur comprise entre 25 et 100 µm et une hauteur de quelques millimètres. Cette fente d'entrée est placée dans le plan focal d'une lentille de collimation ayant une faible aberration et une ouverture numérique d'environ 0,4. Il peut donc être souhaitable de disposer d'un faisceau laser dans la cavité optique ayant, dans la zone de recueil 52, un diamètre tel que son image par le dispositif optique 50 coïncide avec la fente d'entrée 310 du spectromètre 300. Ou inversement, pour optimiser la collecte de la lumière diffusée, il peut être souhaitable que le diamètre du faisceau laser dans la zone de recueil 52 corresponde à l'image par le dispositif optique 50, de la largeur de la fente d'entrée 310.

Selon un mode de réalisation, l'adaptation du diamètre du faisceau dans la zone de recueil 52 dans la cavité optique 180, à la largeur de la fente d'entrée 310 du spectromètre, est réalisée par un choix approprié du rayon de courbure des miroirs de cavité 182, 184, 186. Comme cela est connu, pour une hauteur de fente d'entrée donnée, et une ouverture numérique d'optique d'entrée de spectromètre donnée, le rapport d'agrandissement du dispositif optique 50 peut être déterminé, et les paramètres de focalisation optimums du faisceau laser dans la cavité peuvent être calculés (voir par exemple réf. [1]). Par exemple, si l'on considère les paramètres d'un spectromètre de Raman compact classique, tel que celui portant la référence WP 785L, conçu pour opérer à la longueur d'onde d'excitation de 785 nm, ayant une ouverture numérique de 0,38, et une hauteur de fente d'entrée de 0,6 mm, le diamètre optimal du faisceau laser d'excitation dans la cavité est de 24,5 µm. Le choix d'un tel diamètre permet d'obtenir une intensité maximum pour la lumière de Raman diffusée, et donc pour le signal de Raman. Cela implique que si la fente d'entrée du spectromètre présente une largeur de 25 µm, et si le faisceau laser d'excitation de 785 nm est focalisé à un diamètre de 24,5 µm, alors le signal de Raman qui peut être observé, présente l'intensité la plus élevée possible. Cependant, dans un système classique de spectrométrie, à rétroaction optique verrouillée sur une cavité optique en V, avec trois miroirs de cavité ayant un même rayon de courbure de 400 mm et des bras de cavité optique de 200 mm de longueur, le diamètre du faisceau laser dans la cavité atteint localement au minimum 418 µm, ce qui correspond à 16,7 fois la largeur optimum. Cela implique une perte multipliée par 10 par rapport à ce qui pourrait être obtenu lorsque le couplage est optimum. Si l'on diminue le rayon de courbure des miroirs de cavité 182, 184, 186, par exemple à 20 mm, en plaçant ces derniers de manière à réduire les bras de cavités à 30 mm, le diamètre du faisceau laser dans la cavité optique, vu par le dispositif optique 50, passe localement à une valeur minimum 94 µm, ce qui correspond à 4 fois la largeur optimum. Théoriquement, il serait possible de réduire le rayon de courbure des miroirs de cavité pour réduire localement le diamètre du faisceau laser dans la cavité à la valeur optimum de 24,5 µm, mais cela impliquerait des valeurs irréalistes pour le rayon de courbure des miroirs de cavité (0,7 mm) et pour la longueur de la cavité (1,05 mm).

Selon un mode de réalisation, le diamètre du faisceau laser d'excitation dans la cavité est réduit localement en choisissant trois miroirs de rayons de courbure distincts et des longueurs de bras de cavité en V différentes. Selon un exemple de réalisation, les miroirs 182 et 184 présentent un rayon de courbure fixé à 20 mm et sont espacés de 30,3 mm, et le miroir 186 est plan et distant du miroir d'entrée 182 de 160 mm. Ces dispositions permettent d'obtenir un faisceau laser d'excitation dans la cavité présentant localement un rétrécissement de 26 µm de diamètre. Le rétrécissement du faisceau laser d'excitation est situé à une distance de 20 mm du miroir 184. Ces dispositions permettent d'obtenir un bien meilleur couplage de la lumière diffusée à l'entrée du spectromètre, et donc une bien meilleure sensibilité de mesure.

Pour illustrer le fonctionnement du système d'analyse de la figure 1, les figures 2 à 4 représentent sur une même échelle de temps, des chronogrammes de la forme d'un signal de commande 200 fourni à la source laser 100, un signal 220 issu du photodétecteur 190 (représentatif de l'intensité lumineuse accumulée dans la cavité optique 180), et un signal 240 de contrôle du capteur 330.

Dans la description qui suit, on suppose que la fréquence d'émission de la source laser est commandée par son courant d'alimentation. Bien entendu, si la source laser utilisée est contrôlée par un autre paramètre, les dispositions décrites ci-après du courant d'alimentation s'appliquent également à ce paramètre de commande.

Selon un mode de réalisation, le signal de commande 200 de la source laser 100 est un courant d'alimentation 200 comprenant une composante continue 202, et une composante variable 204, par exemple en dent de scie, qui s'ajoute à la composante continue 202. La composante continue 202 est fixée à une valeur $I_0$ proche du courant nominal pour générer un faisceau laser 121 ayant une puissance proche de la valeur nominale de la source laser 100. La composante variable 204 présente une amplitude de variation $\Delta I$, et des phases ou rampes montantes 205 séparées par des phases ou rampes descendantes 206 de plus courte durée. Durant les phases montantes 205, la longueur d'onde intrinsèque du faisceau laser 121 émis par la source laser 100 (en l'absence de rétroaction) augmente à partir d'une valeur minimum $\lambda_0$ correspondant au courant $I_0$. Durant les phases descendantes 206, la longueur d'onde intrinsèque du faisceau laser 121 diminue pour atteindre la valeur minimum $\lambda_0$.

Selon un mode de réalisation, la durée des phases descendantes 206 est réduite de manière à ce que la fréquence d'émission de la source laser varie suffisamment rapidement durant ces phases pour qu'aucune accumulation de puissance lumineuse n'ait le temps de se former dans la cavité optique 180.

Selon un mode de réalisation, l'amplitude de variation $\Delta I$ du courant d'alimentation de la source laser 100 est définie de manière à atteindre successivement plusieurs modes de résonance consécutifs de la cavité optique 180. Ainsi, le signal 220 qui est obtenu dans une situation où le faisceau de rétroaction présente l'amplitude et la phase requises, présente plusieurs pics d'intensité lumineuse 221, 222, ... 229, pendant chacune des phases montantes 205 du courant d'alimentation 200. En outre, il peut être observé que plus le courant d'alimentation est élevé, plus la puissance optique accumulée dans la cavité est élevée.

L'existence de ces pics 221-229 montre que la source laser 100 se verrouille successivement sur plusieurs modes de résonnance de la cavité 180. Grâce à la mise en oeuvre d'un tel courant d'alimentation de la source laser 100 assurant le balayage d'au moins deux modes de cavité, des pics d'intensité lumineuse apparaissent quelle que soit la valeur de la première longueur d'onde intrinsèque émise par la diode laser, et virtuellement quelle que soit la phase du faisceau de rétroaction optique, à l'exception d'un petit intervalle autour du point où la phase est en opposition à la phase optimum. Il est ainsi possible, à la suite d'une perte soudaine du verrouillage de la phase, de mettre en oeuvre le procédé de restauration suivant.

Lorsque la phase du faisceau de rétroaction est erronée, l'amplitude maximum des pics d'intensité lumineuse dans la cavité reste inférieure à la valeur maximum atteinte lorsque la phase est ajustée à sa valeur optimum. Lorsque l'amplitude maximum observée des pics d'intensité lumineuse chute en dessous d'une valeur de seuil fixée à une valeur inférieure à la valeur maximum atteinte lorsque la phase est ajustée à sa valeur optimum, le processeur CP démarre une rampe couvrant au moins une phase de $4\pi$, et observe le faisceau laser dans la cavité jusqu'à ce que la condition d'accord de phase soit à nouveau détectée. En pratique, la restauration de l'accord de phase dure généralement quelques périodes de la composante variable 204, et le signal de Raman peut être acquis sans interruption.

Dès qu'un des pics 221-229 est observé à l'aide du photodétecteur 190, la phase du faisceau de rétroaction peut être ajustée finement, la valeur optimum de la phase étant atteinte lorsque l'instant d'apparition du sommet d'au moins un des pics 221-229 est situé au milieu de l'intervalle entre les instants de début et de fin du pic. L'ajustement fin de la phase du faisceau de rétroaction peut être effectué en analysant la dérivée du signal 220, l'instant d'apparition des sommets correspondant aux instants où la dérivée du signal 220 est nulle, et les instants de début et de fin des pics correspondants aux instants où la dérivée du signal 220 est maximum.

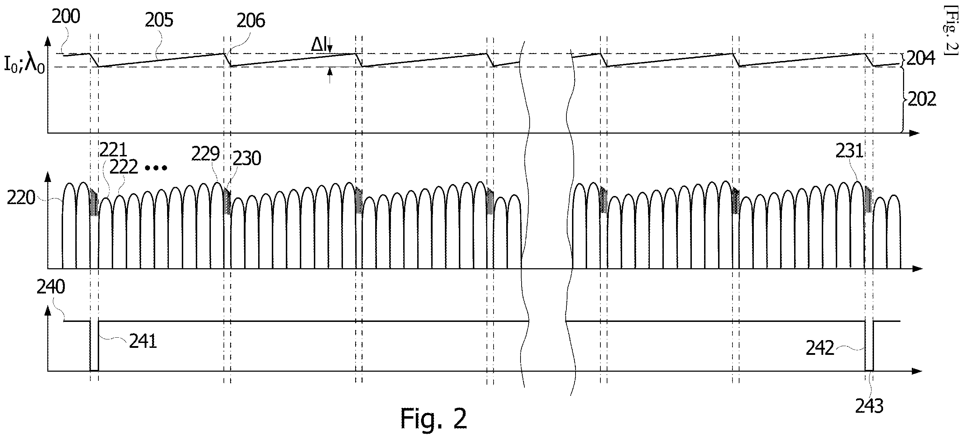

Ensuite, le centrage des pics 221-229 peut être ajusté par rapport aux phases montantes 205 du courant 200 en agissant sur la valeur de la composante constante $I_0$, ou sur la température de la source laser 100 et sur l'amplitude $\Delta I$ de variation du courant 200. L'absence d'un des pics 221-229 (période où la puissance dans la cavité est nulle) risque en effet d'entraîner une certaine instabilité de la puissance lumineuse accumulée dans la cavité. Sur la figure 2, ce centrage est optimum dans la mesure où le démarrage du premier pic 221 coïncide avec le début de la phase montante 205, où la fin du dernier pic 229 coïncide avec la fin de cette phase, et donc où le nombre maximum de pics pendant une phase montante 205 est atteint. En faisant varier la valeur de la composante continue $I_0$ (202), on agit sur l'instant de début du premier pic 221, et en faisant varier l'amplitude $\Delta I$, on agit sur la coïncidence entre l'instant de fin du dernier pic et la fin de la phase montante 205. De telles corrections peuvent en effet être nécessaires notamment en raison du vieillissement de la source laser, entrainant des variations de ses caractéristiques (longueur d'onde du faisceau émis en fonction du courant d'alimentation, ou paramètres caractérisant la rétroaction).

Durant les phases descendantes 206 du courant d'alimentation 200, il se produit une séquence 230 où les modes de résonance sont rapidement balayés, en raison de la durée plus courte des phases descendantes. La puissance accumulée dans chaque mode est donc moindre que durant les phases montantes. Il peut être observé sur la figure 2 que la longueur des phases descendantes 206 a été agrandie à des fins de clarté, pour montrer que durant ces phases, la source laser 100 se verrouille séquentiellement sur les mêmes modes de cavité que durant les phases montantes 205, mais dans un ordre inverse. Selon un mode de réalisation, les phases descendantes 206 sont raccourcies au point que le balayage des modes de cavité est réalisé trop rapidement pour que la source laser puisse se verrouiller sur les modes de cavité. Cette disposition permet d'éviter une pollution du signal de Raman par des signaux transitoires qui pourraient apparaître à cause d'un verrouillage de mode durant les phases descendantes 206, et qui pourraient affecter le processus de verrouillage durant les phases montantes 205. Contrairement aux solutions de l'art antérieur utilisant un courant d'alimentation de la source laser ayant une amplitude de variation définie de manière à atteindre un seul mode de résonance de la cavité, il n'est pas nécessaire de centrer la phase montante du courant d'alimentation sur un mode de résonance, puisque l'amplitude $\Delta I$ de variation du courant d'alimentation de la source laser permet systématiquement d'atteindre plusieurs des modes de la cavité. Il apparaît également que ce procédé d'excitation d'une cavité optique est peu sensible aux perturbations mécaniques, le maintien du verrouillage sur les différents modes de résonance de la cavité étant très robuste. En réalité, le verrouillage de la source laser 100 sur un mode de la cavité optique peut être perdu seulement lorsque le photodétecteur 190 ne détecte pas de signal dans la cavité, en cas de déphasage important du faisceau de rétroaction. Lorsque l'intensité des pics mesurés par le photodétecteur 190 est inférieure à une certaine valeur de seuil, la phase du faisceau de rétroaction est commandée de manière à effectuer un balayage rapide de la valeur minimum à la valeur maximum de la phase, jusqu'à ce que le début des pics devienne visible. Le processus de verrouillage peut alors se produire. Pour cette raison, dans les cas où une perte de verrouillage se produit, la restauration du verrouillage est rapide et aisée.

Il convient de noter que la phase du faisceau de rétroaction peut être ajustée en modifiant la longueur du chemin optique entre la source laser et le miroir de couplage 182, par exemple en commandant un petit déplacement du miroir 153 au moyen du transducteur 154. D'autres moyens connus d'ajustement de la longueur du chemin optique peuvent également être utilisés, par exemple un modulateur électro-optique tel que décrit dans la demande WO 2018/060285.

La valeur optimum de l'amplitude ΔI de variation du courant d'alimentation de la source laser 100 peut être déterminée à partir de l'écart de courant δI nécessaire au passage d'un mode de résonnance à l'autre, et du nombre de modes $N_m$ à exciter souhaité. Ainsi, l'amplitude ΔI peut être obtenue à l'aide de l'équation suivante :

$$\Delta I = N_m \times \delta I + \delta Is \qquad (1)$$

où δIs représente un courant additionnel de stabilité tel que : $0 < \delta Is < \delta I$.

Il peut être observé également que le procédé d'excitation de la cavité optique 180, décrit précédemment, consistant à balayer plus d'un mode de résonnance de la cavité, semble contredire le fait que la spectrométrie de Raman requiert un faisceau laser monochromatique. Si cette condition n'est pas réalisée, la résolution des mesures se trouve dégradée. En effet, si plusieurs modes de résonnance sont excités durant chaque phase 205, 206 du courant d'alimentation 200 de la source laser, l'excitation de la cavité 180 n'est pas monochromatique, mais présente la forme d'un train d'impulsions de différentes longueurs d'onde. Par exemple, si l'on utilise en tant que capteur 330 pour le spectromètre de Raman une barrette CCD (Charge-Coupled Device) de 1024 à 2048 cellules photosensibles (pixels). Un tel capteur peut couvrir une bande de fréquences de Raman, par exemple allant de 2000 à 4000 cm$^{-1}$. Un élément d'image de spectre devrait être fourni par au moins trois pixels du capteur 330. Dans un cas extrême où un élément d'image de spectre correspond à un seul pixel, la largeur spectrale d'un élément de spectre peut ne pas excéder 2 à 4 cm$^{-1}$. Par ailleurs, l'écart de fréquence entre deux modes de résonnance dans une cavité en V ayant des branches de 20 cm est de 0,0125 cm$^{-1}$. Cela signifie qu'il est possible de balayer plusieurs dizaines de modes de résonnance de cavité durant une seule rampe 205 de la composante variable 204 du courant d'alimentation 200 de la source laser 100, sachant que ces modes de résonnance ne peuvent pas être distingués avec une résolution spectrale au mieux de 2 cm$^{-1}$. Il en résulte que le faisceau laser présent dans la cavité 180 pendant une rampe 205 excite des modes de cavité qui restent dans une plage de fréquences de largeur bien inférieure à la résolution du capteur (soit 4 cm$^{-1}$).

Le système d'analyse de gaz décrit précédemment présente une grande stabilité et est capable de maintenir indéfiniment le verrouillage de la source laser, sauf en cas de très forte perturbation mécanique. Dans le cas d'une telle perturbation, la restauration du verrouillage est très rapide (moins d'une seconde). En raison de la puissance élevée susceptible d'être accumulée dans la cavité optique, le spectromètre présente une grande sensibilité et offre ainsi une grande précision dans la détermination de concentrations de gaz présents dans la cavité optique.

Dans le cadre d'un spectromètre de Raman, il est préférable d'utiliser un atténuateur qui transmet toute la puissance du faisceau issu de la source laser 100, tout en atténuant le faisceau de rétroaction avec un facteur d'atténuation ajusté à la valeur optimale. Selon un mode de réalisation, le module d'atténuation 160 comporte module d'isolation de Faraday comportant un polariseur d'entrée 162, un milieu optique de Faraday 164, et un polariseur de sortie 168. Le milieu optique de Faraday 164 est placé dans un champ magnétique, par exemple produit par un aimant permanent 166, colinéaire à la direction de propagation du faisceau issu de la source laser 100. Lorsque la rotation de Faraday appliquée par le milieu optique 164 au faisceau incident provenant du polariseur d'entrée 162 est égale à 45° et correspond à l'orientation du polariseur de sortie 168, le faisceau incident subit une faible perte de puissance. En revanche, le faisceau de rétroaction traversant le module 160 dans la direction inverse se trouve polarisé perpendiculairement à l'axe de transmission du polariseur d'entrée 162. Ainsi, si le milieu optique 164 ne produit pas de dépolarisation, et si les polariseurs sont idéaux, le faisceau de rétroaction est complètement atténué. Autrement-dit, le rapport d'atténuation est nul. Le réglage du rapport d'atténuation est effectué en ajustant l'écart d'angle de polarisation entre les deux polariseurs 162, 168. Ce réglage vise à porter la largeur des pics 221-229 à une valeur optimum, afin de réduire les intervalles d'intensité nulle entre les pics d'intensité lumineuse, ou au moins un de ces intervalles.

Habituellement, ce réglage de l'intensité du faisceau de rétroaction est effectué de temps en temps, par exemple lors d'une phase de démarrage du système. En effet, les propriétés de la cavité et de la source laser peuvent varier lentement avec le temps, conduisant l'intensité du faisceau de rétroaction à s'écarter de sa valeur optimum. Cependant, il peut être souhaitable de pouvoir ajuster dynamiquement l'intensité du faisceau de rétroaction.

Par ailleurs, il s'avère que les propriétés magnétiques des isolateurs de Faraday, telles que le champ magnétique et la constante de Verdet, varient en fonction de la température. La température interne du spectromètre de Raman peut être maintenue constante, Dans un environnement perturbé, le spectromètre peut également subir des changements de température ambiante, qui peuvent impacter la répartition de la température à l'intérieur du spectromètre, même si la température à l'intérieur de la cavité 180 est maintenue constante. En réalité, la température du module d'atténuation 160 peut varier, ce qui peut également impacter l'intensité du faisceau de rétroaction, qui peut s'écarter de sa valeur optimale. Or, une diminution de l'intensité du faisceau de rétroaction peut ainsi entrainer une perte de sensibilité du spectromètre. Par ailleurs, une augmentation de l'intensité du faisceau de rétroaction peut entrainer un élargissement des pics 221-229 au point de dépasser la distance entre les modes de cavité adjacents, et donc provoquer un arrêt du fonctionnement du spectromètre en raison d'une perte de mode de cavité. Une marge de sécurité peut être introduite en fixant l'intensité du faisceau de rétroaction à une valeur inférieure à la valeur optimale. Cependant, cette solution a pour effet de réduire la puissance accumulée dans la cavité et donc la sensibilité du spectromètre.

Selon un mode de réalisation, un dispositif de chauffage est couplé au module d'atténuation 160 pour compenser des variations indésirables de la température du module 160, en se basant sur la largeur des pics d'intensité lumineuse dans la cavité. Le dispositif de chauffage peut être, par exemple une cellule de Peltier ou une bande chauffante entourée autour du module 160. Par ailleurs, l'intensité optimale initiale du faisceau de rétroaction est fixée à une valeur correspondant à une température du module 160 supérieure à la température de consigne de l'intérieur du spectromètre. En outre, le module 160 est configuré pour qu'une isolation maximum soit atteinte à une température supérieure. Avec une telle configuration initiale, la largeur des pics 221-229 d'intensité lumineuse dans la cavité est surveillée à l'aide du signal 220 fourni par le photodétecteur 190. Si la largeur de ces pics 221-229 doit être réduite, la température du module 160 est réduite, en abaissant la tension d'alimentation du dispositif de chauffage, ce qui permet d'augmenter l'intensité du faisceau de rétroaction, et inversement. Ainsi, la commande du dispositif de chauffage peut être asservie pour maintenir la largeur des pics 221-229 à une valeur optimum, afin de réduire au moins un intervalle d'intensité nulle entre les pics d'intensité lumineuse, et de préférence l'ensemble de ces intervalles d'intensité nulle.

Classiquement, le capteur 330 est commandé de manière à enchaîner des phases d'exposition en alternance avec des phases de lecture durant lesquelles des mesures de spectre de fréquence sont fournies par les cellules photosensibles du capteur. Pour éviter des phénomènes de battement, il peut être avantageux de synchroniser le signal de commande de la source laser avec le spectromètre.

Selon un mode de réalisation, la phase d'exposition du capteur 330, c'est-à-dire la phase d'intégration des photons pénétrant dans le capteur 330, est calée sur un nombre entier de périodes 205-206 du courant d'alimentation 200 de la source laser 100. Le démarrage de la phase d'exposition est synchronisé avec le courant d'alimentation 200, par exemple avec le début d'une rampe montante 205 du courant d'alimentation. La figure 2 représente un signal de commande 240 du capteur 330. Des impulsions 241 et 242 du signal 240 commandent respectivement le début et la fin de la phase d'exposition du capteur 330. Le front montant des impulsions 241, 242 est calé sur le début d'une rampe montante 205 du courant d'alimentation 200, et le front descendant des impulsions 241, 242 est calé sur la fin d'une autre rampe montante 205 du courant d'alimentation.

A la fin de la phase d'exposition, les signaux accumulés dans le capteur 330 sont proportionnels à la valeur intégrale de l'intensité de tous les pics d'intensité lumineuse 221-229 observés pendant cette phase (entre le front montant 241 et le front descendant 242 du signal de commande 240), y compris les pics plus courts 230 apparus durant les rampes descendantes 206 du courant d'alimentation 200.

A titre d'exemple, la cavité optique 180 présente des bras de 20 cm de longueur. Les modes de cavité sont alors distants de 0,0125 cm$^{-1}$. La composante variable 204 peut représenter 15% de la composante continue 202 du courant d'alimentation de la source laser 100, ce qui permet de balayer une quarantaine de modes de cavité. Le spectromètre 330 peut comprendre 1024 pixels et couvrir une bande de fréquences de 250 cm$^{-1}$ à 2500 cm$^{-1}$. Sa résolution spectrale est donc de 10 cm$^{-1}$. La quarantaine de modes de cavité balayée représente donc moins d'un quart de la largeur spectrale par pixel. Le fait que la source d'excitation soit non-monochromatique présente donc un effet négligeable sur les performances du spectromètre de Raman, en comparaison avec une source d'excitation purement monochromatique. Il peut être observé que la robustesse du système n'est pas affectée si seulement moins de dix modes de cavité sont balayés. La période de la composante variable 204 peut être comprise entre 0,05 s et 0,2 s, la valeur minimum correspondant à atteinte lorsque la vitesse de balayage est trop élevée et impacte la hauteur des pics d'intensité lumineuse dans la cavité. La durée des phases d'exposition du capteur 330 peut alors être comprise entre 10 et quelques centaines de périodes de la composante variable 204, soit une fraction de seconde à quelques dizaines de secondes, selon l'intensité du signal de Raman. Si la longueur des bras de la cavité optique 180 est réduite à 10 cm, une composante variable 204 de 15% de la composante continue 202 du courant d'alimentation de la source laser 100 permet de balayer une vingtaine de modes de cavité. L'impact de ce balayage sur la résolution spectrale du spectromètre reste cependant inchangé.

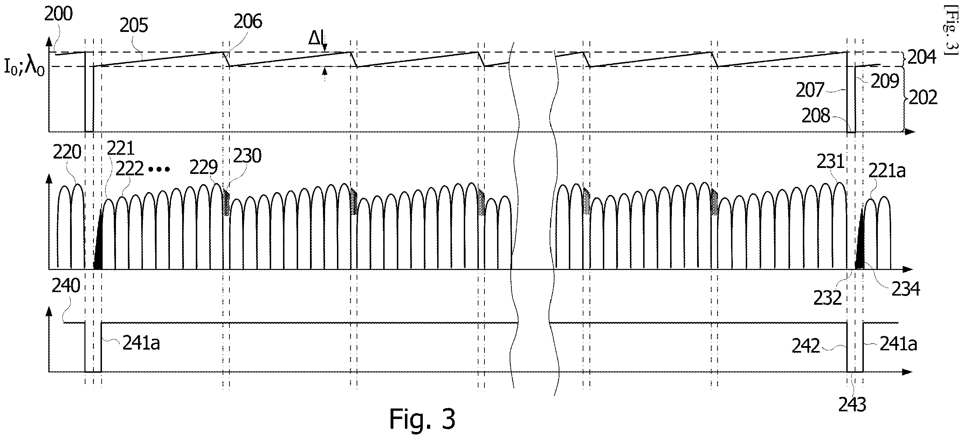

Selon un mode de réalisation, le courant d'alimentation 200 est mis à zéro à la fin de la phase d'exposition du capteur 330, marquée par le front descendant 242 du signal 240. Ainsi, comme illustré sur la figure 3, le courant d'alimentation 200 est mis à zéro pendant la période 208 entre les fronts 207 et 209. Au front montant 209, le courant d'alimentation reprend la valeur $I_0$ (202).

Considérons le cas où la fin de la phase d'exposition du capteur 330 est déclenchée à la fin d'une rampe montante. Pendant le temps 208 où la source laser est éteinte, entre les fronts 207 et 209, une partie 232 du signal 220 fourni par le photodétecteur 190 correspond à une mesure en l'absence de lumière. Cette mesure est mémorisée pour être ensuite soustraite à toutes les mesures du signal 220 fourni par le photodétecteur 190. Ainsi, sur la figure 3, le signal corrigé 220 est nul pendant la période 232 suivant le pic 231, correspondant à la période 208 où la source laser 100 est éteinte. Cette disposition permet de corriger des dérives du photodétecteur 190 qui fournit une mesure de signal noir (dark current) en l'absence de lumière, cette mesure pouvant varier en fonction du temps.

A partir de l'instant 209 où le courant d'alimentation de la source laser 100 est rétabli à la valeur $I_0$, la puissance du faisceau dans la cavité 180 met un certain temps pour s'accumuler, ce qui apparaît sur la forme d'onde 234 dans le signal 220. Ce phénomène résulte de la température de jonction de la source laser 100 qui diminue de quelques degrés en dessous de sa température nominale de fonctionnement, en raison de la coupure de son alimentation. Ainsi, la source laser 100 démarre en émettant une longueur d'onde plus courte que celle du premier pic d'intensité lumineuse 221, puis la longueur d'onde émise augmente jusqu'à atteindre la longueur d'onde correspondant au premier pic d'intensité lumineuse 221, ce qui produit la forme d'onde 234 incluant des excitations successives de modes de résonnance de la cavité, à des longueurs d'onde inférieures à la bande de longueurs d'onde balayée. Il s'avère que de telles excitations peuvent "polluer" les mesures réalisées par le spectromètre.

Selon un mode de réalisation, ce phénomène de pollution résultant de l'extinction de la source laser 200, effectuée pour actualiser la mesure de signal noir du photodétecteur 190, est évité en reportant le début 241 de la phase d'exposition du capteur 330 au début de l'apparition du premier pic 221 (ou 221a), soit à l'instant 241a. Bien entendu, la fréquence des coupures 208 du courant d'alimentation de la source laser 100 est adaptée à la vitesse de variation du signal de noir du photodétecteur 190.

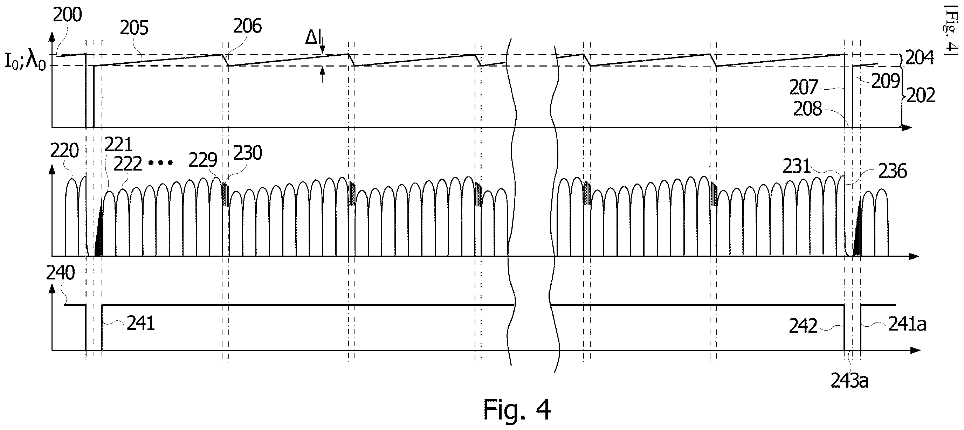

La coupure 208 du courant d'alimentation 200 de la source laser 100 peut intervenir à un moment où un faisceau laser d'une certaine intensité est présent dans la cavité 180. Ce cas est illustré par la figure 4. La puissance accumulée dans la cavité 180 ne peut pas disparaitre instantanément, même si la source laser 100 n'émet plus. En réalité, la puissance du faisceau accumulée dans la cavité 180 diminue exponentiellement jusqu'à disparaître, comme cela est montré par la forme du signal 236, avec une constante de temps de décroissance $\tau$ qui dépend des pertes des miroirs de cavité 182, 184, 186 et de l'absorption des gaz dans la cavité. Lorsque les miroirs présentent une réflectivité très élevée, la constante de temps $\tau$ peut être comprise entre une fraction de microseconde et plusieurs centaines de microsecondes.

Selon un mode de réalisation, la coupure du courant d'alimentation de la source laser 100 est effectuée à l'instant où le dernier pic 231 du signal 220 atteint sa valeur maximum, ce dernier pic se produisant à la fin d'une rampe montante 205 du courant d'alimentation 200. Cette opération permet de d'obtenir des informations sur l'état de la cavité 180. En effet, dans une application à l'analyse de gaz, des poussières ou des produits corrosifs peuvent affecter la réflectivité des miroirs de cavité 182, 184, 186. Il s'avère en effet que le temps de décroissance du signal 220 après coupure du courant d'alimentation 200 est représentatif des pertes dans la cavité et donc de l'état des miroirs de cavité. Si la mesure de ce temps de décroissance est trop élevée, une alarme peut être déclenchée pour signaler qu'un nettoyage des miroirs de cavité est nécessaire. Après un temps d'attente suffisant pour s'assurer que la décroissance est terminée, plusieurs mesures du signal 220 issu du photodétecteur 190 peuvent être acquises, pour déterminer ensuite le courant de noir par un calcul de moyenne de ces mesures. Par ailleurs, les circuits de pixel des capteurs de spectromètre tels que ceux de type CCD produisent également des signaux de noir qui peuvent varier dans le temps et d'un circuit de pixel à l'autre. Ces signaux induisent du bruit qui limite la résolution et la sensibilité du spectromètre de Raman. Le signal de noir de chaque circuit de pixel présente une composante aléatoire qui change à chaque phase d'exposition du capteur 330. Cette composante aléatoire est due principalement au bruit thermique apparaissant dans les cellules sensibles à la lumière des circuits de pixel, et dans les circuits de lecture RDC du capteur 330. Cette composante aléatoire reste cependant assez faible et peut être réduite par un calcul de moyenne proportionnellement à la racine carrée du nombre d'expositions moyennées. Les circuits de pixel de tous les capteurs de spectromètre présentent un courant de noir ayant une composante fixe qui varie d'un pixel à l'autre et qui est liée à la fabrication du capteur 330. Cette composante fixe peut atteindre des valeurs significatives. Les composantes fixes des courants de noir de tous les circuits de pixel d'un capteur de spectromètre forment un motif de bruit fixe ou FPN (Fixed Pattern Noise) qui détériore considérablement la sensibilité du système. Habituellement, le FPN pour un capteur de spectromètre est prédéterminé et préenregistré, et soustrait des signaux de pixels fournis par le capteur 330. Cependant, il s'avère que le FPN d'un capteur évolue dans le temps, notamment en raison du vieillissement des composants du capteur 330. Il est donc nécessaire de procéder périodiquement à des expositions multiples en l'absence de lumière pour calculer des valeurs moyennes. Généralement, le FPN doit être rafraichi avec une périodicité de quelques heures en fonction de la stabilité du système. En spectrométrie de Raman appliquée aux milieux gazeux nécessitant de capter de faibles signaux de lumière diffuse, les temps d'exposition sont généralement longs, de l'ordre de plusieurs dizaines de secondes, et parfois davantage, notamment lorsque les mesures issues de plusieurs expositions sont moyennées. Or il n'est pas souhaitable, en particulier dans des applications de contrôle de processus, d'interrompre l'acquisition de mesures pendant plusieurs minutes pour rafraichir le FPN.

Selon un mode de réalisation, un FPN initial est déterminé lors d'une procédure de démarrage du spectromètre sur la base d'un nombre suffisant d'expositions en l'absence de lumière. Durant l'utilisation du spectromètre, des mesures en plus petit nombre du signal de noir, avec le laser éteint, sont réalisées et combinées aux valeurs du FPN initial ou précédent, par exemple par un calcul de moyenne glissante. Par exemple, la détermination du signal de noir peut être réalisée périodiquement, après plusieurs dizaines de phases d'exposition consécutives pour la mesure de signaux de Raman. Une mesure du signal de noir peut consister à couper l'alimentation de la source laser 100 durant une phase d'arrêt s'étendant sur une ou plusieurs phases d'exposition consécutives. Durant cette phase d'arrêt, une mesure de courant de noir est acquise pour chaque cellule photosensible du capteur de spectromètre 330 par une lecture de la cellule. Chaque mesure est utilisée pour corriger une valeur moyenne de courant de noir mémorisée pour la cellule photosensible correspondante, par exemple par un calcul de moyenne glissante. La valeur moyenne de courant de noir de chaque cellule photosensible est utilisée pour corriger chaque mesure de spectre fournie par la cellule photosensible.

Selon un mode de réalisation, les mesures de courant de noir dans les cellules photosensibles sont précédées de plusieurs mesures successives non prises en compte, réalisées au début de la phase d'arrêt, afin d'effacer les cellules photosensibles, c'est-à-dire afin de s'assurer que les charges électriques accumulées dans les cellules photosensibles durant les phases d'exposition précédant la phase d'arrêt ont bien été évacuées.

Dans la configuration présentée sur la figure 1, la lumière diffusée dans la cavité optique est observée par le dispositif optique 50 sensiblement perpendiculairement à la direction de propagation du faisceau 172, 173 dans la cavité 180. Les dispositions décrites précédemment en référence à la figure 1 s'appliquent également au cas d'une observation coaxiale où la lumière diffusée dans la cavité optique est observée dans l'axe du faisceau 172 ou 174 au travers du miroir 184 ou 186.

Selon un mode de réalisation, l'observation de la lumière diffusée dans la cavité optique 180, est coaxiale, et les deux miroirs de fond de cavité 184, 186 présentent un coefficient de réflexion maximum dans une bande de longueurs d'onde incluant les longueurs d'onde des modes de résonance de cavité excités (pics 221-229), et un coefficient de transmission maximum en dehors de cette bande de longueurs d'onde. Il existe en effet des revêtements appelés "filtres coupe bande" (notch filters) ayant cette propriété. De cette manière, les longueurs d'onde d'excitation de la cavité (lumière d'excitation et lumière diffusée élastiquement dans la cavité optique) sont réfléchies par les miroirs 184, 186, tandis que la lumière diffusée inélastiquement dans la cavité (signaux de Raman incluant les raies Stokes et anti-Stokes) est transmise par ces miroirs vers le spectromètre. Il existe également des revêtements appelés "filtres à arêtes" (edge filters) ayant un coefficient de réflexion maximum jusqu'à une certaine longueur d'onde à partir de laquelle le coefficient de transmission est maximum. Avec un tel revêtement, les longueurs d'onde d'excitation de la cavité sont réfléchies par les miroirs 184, 186, tandis que seules les raies Stokes (ou anti-Stokes) sont transmises par ces miroirs vers le spectromètre. Ce mode de collecte coaxial présente l'avantage de fournir un signal de Raman plus intense. Cependant, dans un système à cavité optique excitée par une diode laser, ce mode de collecte coaxial entraine l'apparition d'une émission spontanée amplifiée ASE (Amplified Spontaneous Emission), susceptible de masquer les signaux de Raman, cette émission résultant de l'utilisation d'une diode laser. L'usage de tels revêtements de miroir permet d'empêcher la résonnance de l'émission spontanée amplifiée.

Pour obtenir des mesures de spectre significatives sur le plan quantitatif, il convient de tenir compte de variations de la puissance optique accumulée dans la cavité dues notamment à la variation de la composition des gaz présents dans la cavité, à la contamination des miroirs de cavité, et à des désalignements d'injection du faisceau laser dans la cavité. A cet effet, on effectue une normalisation des mesures de spectre. Cette normalisation peut être effectuée en fonction de la valeur intégrale des mesures d'intensité lumineuse fournies par le photodétecteur 190, par exemple en divisant chacune des mesures de spectre obtenues à l'issue de la phase d'exposition en fonction de la valeur intégrale des mesures d'intensité lumineuse du faisceau laser dans la cavité optique.

Selon un mode de réalisation, une partie de la lumière diffusée dans la cavité optique, reçue par le dispositif optique 50 est acheminée vers un photodétecteur additionnel pour mesurer les variations de l'intensité lumineuse de la lumière diffusée. Sachant que l'intensité de la lumière diffusée élastiquement est très grande devant celle de la lumière diffusée inélastiquement, les mesures ainsi obtenues représentent essentiellement la lumière diffusée élastiquement. Une valeur intégrale de ces mesures est ensuite déterminée et peut être utilisée pour normaliser les mesures de spectre fournies par les cellules photosensibles du capteur 330, par exemple en divisant chacune de ces mesures par la valeur intégrale. En pratique, la lumière reçue par le photodétecteur additionnel comprend une composante de diffusion élastique (Raleigh) et une composante de diffusion inélastique (Raman). La composante de diffusion de Raman est inférieure de plusieurs ordres de grandeur à la composante de Rayleigh, et peut donc être négligée. Comme les composantes de Rayleigh et de Raman sont collectées dans un même volume, il est possible de déterminer la valeur du flux total de photons à la longueur d'onde d'excitation, par rapport à une valeur de calibration obtenue au moment de la calibration du système, et ainsi obtenir un spectre de Raman normalisé, à la fois indépendant des variations de l'intensité du faisceau d'excitation dans la cavité, et indépendant de désalignements éventuels entre le dispositif de collecte de la lumière diffusée et le faisceau d'excitation dans la cavité. Le lien entre la composante de Rayleigh et la composition des gaz dans la cavité est pris en compte en considérant les concentrations relatives des gaz déterminées à partir du spectre de Raman. Il est à noter que la contribution de la composante de Raman dans la lumière diffusée mesurée peut être compensée en appliquant à la valeur totale de l'intensité de lumière mesurée, un facteur de correction calculé en fonction d'un rapport connu entre les sections efficaces (cross-sections) de Rayleigh et Raman.

La partie de la lumière diffusée transmise au photodétecteur additionnel peut être extraite de la lumière diffusée transmise au capteur 330 à l'aide d'un coupleur en Y (dans le cas d'une observation perpendiculaire ou coaxiale), ou à l'aide d'une fibre optique dédiée couplée à la sortie du dispositif optique 50 (dans le cas d'une observation perpendiculaire).

Selon un mode de réalisation, les valeurs intégrales obtenues pour la photodiode 190 et pour le photodétecteur additionnel sont comparées, par exemple en calculant le rapport entre ces deux valeurs intégrales, pour déterminer un éventuel désalignement du dispositif optique 50 avec le faisceau laser dans la cavité optique, En comparant ce rapport à une valeur de seuil, il est possible de déterminer lorsque ce désalignement est excessif.

Il est ainsi possible de surveiller en permanence l'évolution des paramètres de fonctionnement du système, de corriger en temps réel des dérives, sans avoir à interrompre les mesures de gaz dans la cavité, et de signaler lorsqu'il n'est plus possible d'obtenir des mesures fiables. Il en résulte que le système d'analyse de gaz précédemment décrit peut être utilisé dans le cadre d'applications critiques, telles que le contrôle de processus industriels, notamment dans le secteur pétrolier, et le contrôle de gaz anesthésiants dans le secteur médical, ou dans des environnements très perturbés (vibrations, variations de température ambiante).

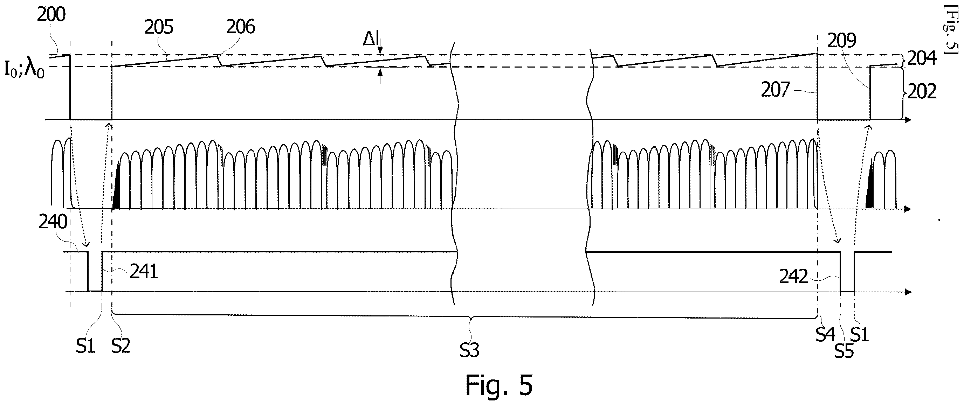

En général, l'acquisition des signaux par le capteur 330 est contrôlée par un processeur distinct possédant sa propre horloge. Il en résulte que le début et la fin des phases d'exposition peuvent intervenir à des moments arbitraires du signal de commande 200. Cette absence de synchronisation peut conduire à des incohérences entre le signal de référence intégré fourni par le photodétecteur 190 et les quantités de lumière diffusée accumulées dans le capteur 33 pendant les phases d'exposition. Ces incohérences peuvent affecter de manière significative la qualité de la normalisation du signal de Raman. Cette détérioration de la qualité de la normalisation peut être évitée en établissant une certaine liaison entre les phases d'exposition et phases montantes et descendantes du signal de commande 200, et en mettant en oeuvre la procédure suivante, illustrée par la figure 5.Selon cette procédure, la durée de la phase d'exposition du capteur 330 est fixée à une valeur égale à un nombre entier Nrp de périodes du signal de commande 200, plus une fraction de cette période. Avant de démarrer la phase d'exposition (accumulation de la lumière de Raman dans le capteur 330), le signal de commande 200 est maintenu à zéro. Il est ainsi assuré qu'aucune lumière n'est présente dans la cavité 180. Au début d'une étape S 1, un signal de démarrage de la phase d'exposition 241 est envoyé à une unité de contrôle du capteur 330. Une étape S2 est déclenchée après un certain délai permettant d'assurer que l'exposition du capteur 330 a démarré. A l'étape S2, la source laser 100 est allumée, en lui appliquant le signal de commande périodique 200. Nrp périodes du signal de commande 200 sont appliquées à la source laser pendant une étape S3. Le capteur 330 reçoit alors le signal de Raman. La fin de la période Nrp du signal de commande 200, marque la fin de l'étape S3 et le début de l'étape S4. A l'étape S4, le signal de commande 200 est mis à zéro. La phase d'exposition du capteur 330 se poursuit pendant la fraction de la période du signal de commande 200, jusqu'à une étape S5, tandis qu'aucune lumière de Raman n'est présente dans la cavité. Durant les étapes S1 à S5, le signal issu du photodétecteur 190 est intégré, jusqu'à la réception d'un signal de fin de la phase d'exposition du capteur 330. Cette procédure permet d'assurer que le signal de Raman accumulé dans le capteur 330 est proportionnel au signal intégré issu du photodétecteur 190, et est normalisé sans biais. La réception du signal 242 marquant la fin de la phase d'exposition à l'étape S5 déclenche une nouvelle étape S 1.

Il apparaîtra clairement à l'homme de l'art que la présente invention est susceptible de diverses variantes de réalisation et diverses applications. En particulier, les différents composants optiques décrits précédemment ont été présentés à titre d'exemple et dépendent essentiellement des caractéristiques du faisceau laser émis par la source laser et donc du choix de la source laser. Il va de soi que si les caractéristiques de ce faisceau sont différentes, certains des composants optiques décrits peuvent être inutiles, voire inappropriés, et d'autres composants optiques peuvent être nécessaires. Il importe simplement que les caractéristiques du faisceau laser en entrée de la cavité optique se rapprochent autant que possible de caractéristiques idéales, à savoir une section circulaire (excentricité nulle) et des divergences dans toutes les directions correspondant à celle du mode fondamental de la cavité optique. En effet, la définition des moyens permettant d'atteindre ces caractéristiques entre totalement dans le champ des techniques classiques de conception des systèmes optiques. En outre, la source laser peut être conçue pour produire un faisceau laser ayant des caractéristiques proches ou correspondant aux caractéristiques idéales, de sorte que l'un ou l'autre, voire l'ensemble des modules optiques 110, 120 et 130 ne soient pas nécessaires.

De même, d'autres dispositifs connus pour ajuster la phase et l'intensité d'un faisceau lumineux peuvent être utilisés pour ajuster la phase et l'intensité du faisceau de rétroaction. Par ailleurs, il peut être envisagé de transmettre le faisceau laser issu de la source laser et le faisceau de rétroaction par des chemins optiques différents entre la source laser et l'entrée de la cavité. Cette disposition permet notamment de simplifier le dispositif d'ajustement de l'intensité du faisceau de rétroaction, qui n'a alors pas besoin de pouvoir être traversé par le faisceau laser issu de la source laser, sans agir sur ce dernier.

En outre, il est clair pour l'homme du métier que l'usage d'une cavité en V n'est pas nécessaire. Par exemple, une cavité linéaire à deux miroirs associée aux dispositions décrites dans les références [4] et [5] pour mettre en oeuvre une rétroaction optique, ou à une rétroaction optique verrouillée par un décalage spatial de la diode laser, telle que décrite dans le brevet US 5 835 522, peut être mise en oeuvre dans le cadre de la présente invention.

Références citées

[1] "An Efficient Intracavity Laser Raman Spectrometer", M. Hercher et. al., Applied Spectroscopy 32, No 3, pp 298-301 (1978) ;
[2] "Frequency Stabilization of Semi-conductor lasers by resonant optical feedback", Dahmani et al., Opt. Lett., 12, pp. 876-878 (1987);
[3] "Optically Self-Locked Semiconductor Laser with Servo Control for Feedback Phase and Laser Current", Peter Buch et al., IEEE Journal of Quantum Electronics, vol. 27 (7), pp. 1863-1868 (1991);
[4] "Cavity-enhanced resonant photoacoustic spectroscopy with optical feedback cw diode lasers: A novel technique for ultratrace gas analysis and high-resolution spectroscopy", M. Hippler, C. Mohr, K. A. Keen, E. D. McNaghten, The Journal of Chemical Physics 133, 044308 (2010);
[5] "Cavity-Enhanced Raman Spectroscopy of Natural Gas with Optical Feedback cw-Diode Lasers", M. Hippler, Anal. Chem., 87, 7803-7809 (2015).

## Revendications

1. Procédé d'analyse de gaz par spectrométrie de Raman, le procédé comprenant des étapes consistant à :

   appliquer un signal de commande à une source laser (100) afin qu'elle produise un faisceau laser balayant une plage de fréquences incluant des fréquences de plusieurs modes de résonance d'une cavité optique (180) incluant des gaz à analyser, la largeur de la plage de fréquence étant inférieure à la résolution d'un spectromètre de Raman (300),
   fournir le faisceau laser à une entrée (182) de la cavité optique,
   extraire de la cavité optique un faisceau de rétroaction,
   ajuster une phase et une amplitude du faisceau de rétroaction,
   envoyer le faisceau de rétroaction ajusté en phase et en amplitude, sur la source laser,
   durant le balayage de la plage de fréquences, mesurer des variations d'intensité lumineuse du faisceau laser dans la cavité optique, et détecter des pics d'intensité lumineuse (221-229) dans les variations d'intensité lumineuse, chaque pic d'intensité lumineuse correspondant à un mode de résonance de la cavité optique, la phase du faisceau de rétroaction étant ajustée pour réduire un écart entre un instant médian et un instant d'apparition d'un sommet, d'un des pics d'intensité lumineuse, l'amplitude du faisceau de rétroaction étant ajustée pour réduire au moins un intervalle d'intensité nulle entre les pics d'intensité lumineuse, et
   acquérir à l'aide d'un capteur (330) du spectromètre de Raman (300), des mesures de spectre de lumière diffusée inélastiquement sous l'effet du faisceau laser présent dans la cavité optique, pour déterminer la nature et/ou la concentration des gaz présents dans la cavité optique.

2. Procédé selon la revendication 1, dans lequel :

   le signal de commande de la source laser (100) présente une forme périodique, et
   le capteur (330) du spectromètre de Raman (300) comprenant une pluralité de cellules photosensibles, reçoit une partie de la lumière diffusée inélastiquement et est commandé de manière à enchaîner des phases d'exposition en alternance avec des phases de lecture durant lesquelles les mesures de spectre sont fournies par les cellules photosensibles,
   le procédé comprenant, durant chaque phase d'exposition, des étapes consistant à :

      obtenir une valeur intégrale de mesures d'intensité lumineuse du faisceau laser dans la cavité optique, et/ou d'intensité lumineuse d'une partie de la lumière diffusée élastiquement sous l'effet du faisceau laser présent dans la cavité optique, et
      normaliser chacune des mesures de spectre obtenues à l'issue de la phase d'exposition en fonction de la valeur intégrale, pour obtenir un spectre normalisé.

3. Procédé selon la revendication 2, dans lequel les phases d'exposition présentent une durée égale à un multiple de la période du signal de commande de la source laser, plus une fraction de cette période, le début de chaque phase d'exposition étant synchronisé avec le signal de commande (200) de la source laser (100).

4.  Procédé selon la revendication 2 ou 3, dans lequel chaque phase d'exposition est démarrée et interrompue durant des périodes où aucune lumière n'est présente dans la cavité optique (180).

5.  Procédé selon l'une des revendications 2 à 4, comprenant des étapes consistant à :

    couper périodiquement l'alimentation de la source laser, pour mesurer un courant de noir dans un photodétecteur (190) fournissant les mesures de variation d'intensité lumineuse du faisceau laser dans la cavité optique, et corriger les mesures de variations d'intensité lumineuse du faisceau laser dans la cavité optique, à l'aide d'une moyenne de mesures de courant de noir.

6.  Procédé selon une revendication 5, dans lequel les coupures de l'alimentation de la source laser sont effectuées en dehors des phases d'exposition.

7.  Procédé selon l'une des revendications 2 à 6, comprenant des étapes consistant à :

    couper l'alimentation de la source laser durant une phase d'arrêt s'étendant sur au moins une phase d'exposition consécutive, et
    pour chaque cellule photosensible du capteur (330) du spectromètre de Raman (300) :

    mesurer un courant de noir durant la phase d'arrêt,
    corriger un courant de noir moyen mémorisé pour la cellule photosensible en fonction de la mesure de courant de noir obtenue pour la cellule photosensible, et
    corriger chaque mesure de spectre fournie par la cellule photosensible en fonction du courant de noir moyen mémorisé pour la cellule photosensible,

    la mesure de courant de noir pouvant être précédée de plusieurs mesures non prises en compte pour effacer les cellules photosensibles.

8.  Procédé selon l'une des revendications 1 à 7, dans lequel le faisceau laser fourni en entrée de la cavité optique (180) et le faisceau de rétroaction sont transmis par un même chemin optique entre la source laser et l'entrée de la cavité optique.

9.  Procédé selon l'une des revendications 1 à 8, dans lequel l'amplitude du faisceau de rétroaction est ajustée par un module d'isolation de Faraday (160) ajustable installé sur le chemin optique entre la source laser (100) et l'entrée (182) de la cavité optique (180), le module d'isolation de Faraday atténuant l'amplitude du faisceau de rétroaction et laissant passer le faisceau laser issu de la source laser (100).

10.  Procédé selon la revendication 9, dans lequel le module d'isolation de Faraday (160) est asservi en fonction des mesures d'intensité lumineuse du faisceau laser dans la cavité optique (180), pour minimiser les intervalles d'intensité nulle entre les pics d'intensité lumineuse (221-229), l'asservissement du module d'isolation de Faraday pouvant être réalisé en agissant sur sa température.

11.  Dispositif d'analyse de gaz par spectrométrie de Raman, comprenant :

    une source laser (100),
    une cavité optique (180) pour recevoir des gaz à analyser et comportant une entrée (182) par laquelle est introduit un faisceau laser émis par la source laser (100),
    un organe de commande produisant un signal de commande (200) de la source laser,
    un composant (182) pour extraire un faisceau de rétroaction de la cavité optique,
    des composants (153, 154, 160, 150) pour ajuster le faisceau de rétroaction en phase et en amplitude et pour envoyer le faisceau de rétroaction ajusté en phase et en amplitude sur la source laser,
    un organe de mesure (190) de variations d'intensité lumineuse (220) du faisceau laser dans la cavité, et
    un spectromètre de Raman (300) pour acquérir des mesures de spectre de lumière diffusée sous l'effet du faisceau laser présent dans la cavité optique,
    le dispositif d'analyse étant configuré pour mettre en oeuvre le procédé selon l'une des revendications 1 à 10.

**12.** Dispositif d'analyse de gaz selon la revendication 11, dans lequel la source laser (100) est configurée pour balayer la plage de fréquences sans saut de mode.

**13.** Dispositif d'analyse de gaz selon la revendication 12, dans lequel la cavité optique est en V et comprend un miroir de couplage (182) formant l'entrée de la cavité optique, et deux miroirs de fond de cavité (184, 186), les miroirs de fond de cavité présentant une réflectivité plus grande ou identique à celle du miroir de couplage.

**14.** Dispositif d'analyse de gaz selon la revendication 13, dans lequel les miroirs (182, 184, 186) formant la cavité optique (180) présentent des rayons de courbure adaptés pour ajuster la forme du faisceau à l'intérieur de la cavité optique en une zone (52) de collecte de la lumière diffusée dans la cavité sous l'effet du faisceau laser.

**15.** Dispositif d'analyse de gaz selon l'une des revendications 13 ou 14, dans lequel la lumière diffusée dans la cavité optique est observée au travers de l'un des miroirs de fond de cavité (184, 186), les miroirs de fond de cavité (184, 186) présentant un revêtement ayant un coefficient de réflexion maximum pour le faisceau réfléchi dans chacun des modes de résonance de la cavité optique, et ayant un coefficient de transmission maximum pour au moins une raie de Stockes des raies de Raman.

**16.** Dispositif d'analyse de gaz selon l'une des revendications 11 à 15, dans lequel la source laser (100) est de l'un des types suivants :

une diode laser à rétroaction répartie,
une diode laser à réflecteur de Bragg distribué,
une diode de type Fabry-Perot stabilisée par un réseau de diffraction de Bragg en volume ou holographique, ou par un filtre à bande étroite,
un laser associé à un dispositif d'ajustement de la fréquence d'émission,
un laser à émission de surface et à cavité verticale, couplé à un miroir à membrane diélectrique.

**17.** Dispositif d'analyse de gaz selon l'une des revendications 11 à 16, comprenant un actionneur (154) couplé à un miroir (153) pour modifier une longueur de chemin optique entre la source laser (100) et l'entrée (182) de la cavité optique (180), afin d'ajuster la phase du faisceau de rétroaction.

**Patentansprüche**

**1.** Verfahren zum Analysieren von Gas durch Raman-Spektroskopie, das Verfahren umfassend Schritte, bestehend aus:

Anlegen eines Steuersignals an eine Laserquelle (100), damit sie einen Laserstrahl erzeugt, der einen Frequenzbereich abtastet, der Frequenzen mehrerer Resonanzmodi eines optischen Resonators (180) einschließt, der zu analysierende Gase einschließt, wobei die Breite des Frequenzbereichs geringer als die Auflösung eines Raman-Spektrometers (300) ist,
Bereitstellen des Laserstrahls an einem Eingang (182) des optischen Resonators,
Extrahieren eines Rückkopplungsstrahls aus dem optischen Resonator,
Anpassen einer Phase und einer Amplitude des Rückkopplungsstrahls,
Senden des phasen- und amplitudenangepassten Rückkopplungsstrahls an die Laserquelle,
während des Abtastens des Frequenzbereichs, Messen von Lichtintensitätsschwankungen des Laserstrahls in dem optischen Resonator und Erfassen von Lichtintensitätspeaks (221-229) in den Lichtintensitätsschwankungen, wobei jeder Lichtintensitätspeak einem Resonanzmodus des optischen Resonators entspricht, wobei die Phase des Rückkopplungsstrahls zum Verringern einer Abweichung zwischen einem mittleren Zeitpunkt und einem Zeitpunkt, zu dem ein Scheitelpunkt von einem der Lichtintensitätspeaks auftritt, angepasst wird, wobei die Amplitude des Rückkopplungsstrahls zum Verringern mindestens eines Nullintensitätsintervalls zwischen den Lichtintensitätspeaks angepasst wird, und
Sammeln mithilfe eines Sensors (330) des Raman-Spektrometers (300) von Spektralmessungen von Licht, das unter der Wirkung des Laserstrahls, der in dem optischen Resonator vorliegt, inelastisch gestreut wird, zum Bestimmen der Art und/oder der Konzentration der Gase, die in dem optischen Resonator vorliegen.

**2.** Verfahren nach Anspruch 1, wobei:

das Steuersignal der Laserquelle (100) eine periodische Form aufweist, und der Sensor (330) des Raman-Spektrometers (300), umfassend eine Vielzahl von fotoempfindlichen Zellen, einen Teil des unelastisch gestreuten Lichts aufnimmt und gesteuert wird, um die Belichtungsphasen abwechselnd mit Auslesephasen aneinanderzuhängen, während denen die Spektralmessungen durch die fotoempfindlichen Zellen bereitgestellt werden,

das Verfahren umfassend, während jeder Belichtungsphase, Schritte, bestehend aus: Erhalten eines Integralwerts von Messungen der Lichtintensität des Laserstrahls in den optischen Resonator und/oder der Lichtintensität eines Teils des Lichts, das unter der Wirkung des Laserstrahls, der in dem optischen Resonator vorliegt, elastisch gestreut wird, und

Normalisieren jeder der Spektralmessungen, die am Ende der Belichtungsphase erhalten werden, in Abhängigkeit von dem Integralwert, zum Erhalten eines normalisierten Spektrums.

3. Verfahren nach Anspruch 2, wobei die Belichtungsphasen eine Dauer aufweisen, die gleich einem Vielfachen der Periode des Steuersignals der Laserquelle plus einem Anteil dieser Periode ist, wobei der Anfang jeder Belichtungsphase mit dem Steuersignal (200) der Laserquelle (100) synchronisiert ist.

4. Verfahren nach Anspruch 2 oder 3, wobei jede Belichtungsphase während Perioden, in denen kein Licht in dem optischen Resonator (180) vorliegt, begonnen und unterbrochen wird.

5. Verfahren nach einem der Ansprüche 2 bis 4, umfassend Schritte, bestehend aus:

periodischem Trennen der Stromversorgung der Laserquelle zum Messen eines Dunkelstroms in einen Fotodetektor (190), der die Messungen der Lichtintensitätsschwankung des Laserstrahls in dem optischen Resonator bereitstellt, und

Korrigieren von Messungen der Lichtintensitätsschwankungen des Laserstrahls in dem optischen Resonator mittels eines Durchschnitts von Dunkelstrommessungen.

6. Verfahren nach Anspruch 5, wobei die Stromversorgungstrennungen der Laserquelle außerhalb der Belichtungsphase vorgenommen werden.

7. Verfahren nach einem der Ansprüche 2 bis 6, umfassend Schritte, bestehend aus:

Trennen der Stromversorgung der Laserquelle während einer Anhaltephase, die sich über mindestens eine nachfolgende Belichtungsphase erstreckt, und

für jede fotoempfindliche Zelle des Sensors (330) des Raman-Spektrometers (300):

Messen eines Dunkelstroms während der Anhaltephase,
Korrigieren eines gespeicherten durchschnittlichen Dunkelstroms für die fotoempfindliche Zelle in Abhängigkeit von der erhaltenen Dunkelstrommessung für die fotoempfindliche Zelle, und
Korrigieren jeder Spektralmessung, die durch die fotoempfindliche Zelle bereitgestellt wird, in Abhängigkeit von dem gespeicherten durchschnittlichen Dunkelstrom für die fotoempfindliche Zelle,
wobei dem Messen des Dunkelstroms mehrere Messungen vorangehen können, die zum Löschen der fotoempfindlichen Zellen nicht berücksichtigt werden.

8. Verfahren nach einem der Ansprüche 1 bis 7, wobei der Laserstrahl, der an dem Eingang des optischen Resonators (180) bereitgestellt wird, und der Rückkopplungsstrahl durch einen gleichen optischen Weg zwischen der Laserquelle und dem Eingang des optischen Resonators übertragen werden.

9. Verfahren nach einem der Ansprüche 1 bis 8, wobei die Amplitude des Rückkopplungsstrahls durch ein anpassbares Faraday-Isolationsmodul (160) angepasst wird, das auf dem optischen Weg zwischen der Laserquelle (100) und dem Eingang (182) des optischen Resonators (180) installiert ist, wobei das Faraday-Isolationsmodul die Amplitude des Rückkopplungsstrahls dämpft und den Laserstrahl von der Laserquelle (100) passieren lässt.

10. Verfahren nach Anspruch 9, wobei das Faraday-Isolationsmodul (160) in Abhängigkeit von den Messungen der Lichtintensität des Laserstrahls in dem optischen Resonator (180) zum Minimieren der Nullintensitätsintervalle zwischen den Lichtintensitätspeaks (221-229) reguliert wird, wobei das Regulieren des Faraday-Isolationsmoduls ausgeführt werden kann, indem auf seine Temperatur reagiert wird.

11. Vorrichtung zum Analysieren von Gas durch Raman-Spektrometrie, umfassend:

eine Laserquelle (100),

einen optischen Resonator (180) zum Aufnehmen von zu analysierenden Gasen und der einen Eingang (182) vorweist, durch den ein Laserstrahl eingeführt wird, der durch die Laserquelle (100) emittiert wird,

ein Steuerungselement, das ein Steuersignal (200) der Laserquelle erzeugt, eine Komponente (182) zum Extrahieren eines Rückkopplungsstrahls aus dem optischen Resonator,

Komponenten (153, 154, 160, 150) zum Anpassen der Phasen und der Amplitude des Rückkopplungsstrahls und zum Senden des phasen- und amplitudenangepassten Rückkopplungsstrahls an die Laserquelle,

ein Element (190) zum Messen der Schwankungen der Lichtintensität (220) des Laserstrahls in dem Resonator, und

ein Raman-Spektrometer (300) zum Sammeln von Spektralmessungen des Lichts, das unter der Wirkung des Laserstrahls in dem optischen Resonator gestreut wird, wobei die Analysevorrichtung zum Implementieren des Verfahrens nach einem der Ansprüche 1 bis 10 konfiguriert ist.

12. Gasanalysevorrichtung nach Anspruch 11, wobei die Laserquelle (100 ) zum Abtasten des Frequenzbereichs ohne Modensprung konfiguriert ist.

13. Gasanalysevorrichtung nach Anspruch 12, wobei der optische Resonator V-förmig ist und einen Koppelspiegel (182), der den Eingang des optischen Resonators ausbildet, und zwei Endspiegel (184, 186) umfasst, wobei die Endspiegel eine Reflektivität größer als oder gleich der des Koppelspiegels aufweisen.

14. Gasanalysevorrichtung nach Anspruch 13, wobei die Spiegel (182, 184, 186), die den optischen Resonator (180) ausbilden, Krümmungsstrahlung aufweisen, die zum Anpassen der Form des Strahls im Inneren des optischen Resonators in einer Zone (52) zum Einsammeln des Lichts, das in dem Resonator unter der Wirkung des Laserstrahls gestreut wird, geeignet ist.

15. Gasanalysevorrichtung nach einem der Ansprüche 13 oder 14, wobei das gestreute Licht in dem optischen Resonator durch einen der Endspiegel (184, 186) beobachtet wird, wobei die Endspiegel (184,186) eine Beschichtung aufweisen, die einen maximalen Reflexionskoeffizienten für den Strahl besitzt, der in jedem der Resonanzmodi des optischen Resonators reflektiert wird, und einen maximalen Übertragungskoeffizienten für mindestens eine Stokes-Linie von Raman-Linien besitzt.

16. Gasanalysevorrichtung nach einem der Ansprüche 11 bis 15, wobei die Laserquelle (100) von einer der folgenden Arten ist:

einer Laserdiode mit verteilter Rückkopplung,

einer Laserdiode mit verteiltem Bragg-Reflektor,

einer Diode der Art Fabry-Perot, die durch ein volumetrisches oder holographisches Bragg-Beugungsgitter oder durch ein Schmalbandfilter stabilisiert ist,

einem Laser, der einer Emissionsfrequenzanpassungsvorrichtung zugeordnet ist,

einem Laser mit Oberflächenemission und mit einem vertikalen Resonator, der mit einem Spiegel mit dielektrischer Membran gekoppelt ist.

17. Gasanalysevorrichtung nach einem der Ansprüche 11 bis 16, umfassend einen Aktuator (154), der mit einem Spiegel (153) zum Modifizieren einer Länge des optischen Wegs zwischen der Laserquelle (100) und dem Eingang (182) des optischen Resonators (180) gekoppelt ist, um die Phase des Rückkopplungsstrahls anzupassen.

**Claims**

1. A method for analyzing gas by Raman spectrometry, the method comprising the steps of:

applying a control signal to a laser source (100) to produce a laser beam sweeping a range of frequencies including frequencies of multiple resonant modes of an optical cavity (180) holding gases to be analyzed, the width of the frequency range being less than the resolution of a Raman spectrometer,

supplying the laser beam to an entrance (182) of the optical cavity,

extracting a feedback beam from the optical cavity,

adjusting a phase and an amplitude of the feedback beam,
sending the phase and amplitude adjusted feedback beam to the laser source,
during sweeping of the frequency range, measuring light intensity variations of the laser beam in the optical cavity, and detecting light intensity peaks (221-229) in the light intensity variations, each light intensity peak corresponding to a resonance mode of the optical cavity, the phase of the feedback beam being adjusted to reduce a deviation between a median time and the time of occurrence of an apex of one of the light intensity peaks, the amplitude of the feedback beam being adjusted to reduce at least an interval of zero intensity between the light intensity peaks, and
acquiring, with a spectrometer sensor (330), measurements of the spectrum of light inelastically scattered by the laser beam in the optical cavity to determine the nature and/or concentration of gases in the optical cavity.

2. The method of claim 1, wherein:

the control signal of the laser source (100) has a periodic shape, and
the sensor (330) of the Raman spectrometer (300), comprising a plurality of photosensitive cells, receives a portion of the inelastically scattered light and is controlled to operate exposure phases alternating with readout phases during which spectrum measurements are produced by the photosensitive cells,
the method comprising, during each exposure phase, the following steps:

obtaining an integral value of measurements of light intensity of the laser beam in the optical cavity, and/or of light intensity of a portion of the light elastically scattered by the laser beam in the optical cavity, and normalizing each of the spectrum measurements obtained at the end of the exposure phase as a function of the integral value, to produce a normalized spectrum.

3. The method of claim 2, wherein the exposure phases have a duration equal to a multiple of the period of the control signal of the laser source, plus a fraction of that period, the start of each exposure phase being synchronized with the control signal (200) of the laser source (100).

4. The method of claim 2 or 3, wherein each exposure phase is started and interrupted during periods when no light is present in the optical cavity (180).

5. The method of any of claims 2 to 4, comprising the steps of:

periodically turning off power to the laser source to measure a dark current in a photodetector (190) providing the measurements of light intensity variations of the laser beam in the optical cavity, and
correcting the measurements of light intensity variations of the laser beam in the optical cavity using an average of the dark current measurements.

6. The method of claim 5, wherein the power interruptions of the laser source are performed outside the exposure phases.

7. The method of any of claims 2-6, comprising the steps of:

turning off the power to the laser source during an interruption phase extending over at least one consecutive exposure phase, and
for each light sensitive cell of the sensor (330) of the Raman spectrometer (300):

measuring a dark current during the interruption phase,
correcting a stored average dark current for the photosensitive cell based on the dark current measurement obtained for the photosensitive cell, and
correcting each spectrum measurement provided by the photosensitive cell according to the stored average dark current for the photosensitive cell,

wherein the measurement of dark current may be preceded by several measurements not taken into account to erase the photosensitive cells.

8. The method of any of claims 1 to 7, wherein the laser beam provided at the entrance of the optical cavity (180) and the feedback beam are transmitted through the same optical path between the laser source and the entrance of the optical cavity.

9. The method of any of claims 1 to 8, wherein the amplitude of the feedback beam is adjusted by an adjustable Faraday isolation module (160) installed in the optical path between the laser source (100) and the entrance (182) of the optical cavity (180), the Faraday isolation module attenuating the amplitude of the feedback beam and passing the laser beam from the laser source (100).

10. The method of claim 9, wherein the Faraday isolation module (160) is servo-controlled as a function of light intensity measurements of the laser beam in the optical cavity (180), to minimize the zero intensity intervals between light intensity peaks (221-229), wherein the servocontrol of the Faraday isolation module can be achieved by acting on its temperature.

11. Device for analyzing gas by Raman spectrometry, comprising:

a laser source (100),
an optical cavity (180) for receiving gases to be analyzed and including an entrance (182) into which a laser beam emitted by the laser source (100) is introduced,
a control element providing a control signal (200) of the laser source,
a component (182) for extracting a feedback beam from the optical cavity,
components (153, 154, 160, 150) for adjusting the feedback beam in phase and amplitude and for sending the feedback beam adjusted in phase and amplitude to the laser source,
an element (190) for measuring variations of intensity (220) of the laser beam in the cavity, and
a Raman spectrometer (330) for acquiring spectral measurements of light diffused under the effect of the laser beam present in the optical cavity,
wherein the analysis device is configured to carry out the method according to one of claims 1 to 10.

12. The gas analysis device of claim 11, wherein the laser source (100) is configured to sweep the frequency range without mode hopping.

13. The gas analysis device of claim 12, wherein the optical cavity is V-shaped and comprises a coupling mirror (182) forming the entrance to the optical cavity, and two cavity end mirrors (184, 186), the cavity end mirrors having a reflectivity greater than or the same as the coupling mirror.

14. The gas analysis device of claim 13, wherein the mirrors (182, 184, 186) forming the optical cavity (180) have radii of curvature adapted to adjust the shape of the beam within the optical cavity to a zone (52) for collecting light scattered in the cavity under the effect of the laser beam.

15. The gas analysis device of claim 13 or 14, wherein light scattered in the optical cavity is observed through one of the cavity end mirrors (184, 186), the cavity end mirrors (184, 186) having a coating with a maximum reflectance for the reflected beam in each of the resonance modes of the optical cavity, and having a maximum transmittance for at least one Stokes line of the Raman lines.

16. The gas analysis device of any of claims 11 to 15, wherein the laser source (100) is one of the following types:

a distributed feedback laser diode,
a distributed Bragg reflector laser diode,
a Fabry-Perot type diode stabilized by a volume or holographic Bragg diffraction grating, or by a narrow band filter,
a laser associated with a device for adjusting the emission frequency,
a surface emitting laser with a vertical cavity, coupled to a dielectric membrane mirror.

17. The gas analysis device of any of claims 11 to 16, comprising an actuator (154) coupled to a mirror (153) for altering an optical path length between the laser source (100) and the entrance (182) to the optical cavity (180), to adjust the phase of the feedback beam.

[Fig. 1]

Fig. 1

[Fig. 2]

Fig. 2

[Fig. 3]

Fig. 3

Fig. 4

[Fig. 4]

EP 3 911 942 B1

[Fig. 5]

Fig. 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5432610 A
- US 5642375 A
- US 5684623 A
- US 5835522 A
- US 6222860 B
- WO 03031949 A
- WO 2018060285 A


**Littérature non-brevet citée dans la description**

- **M. HERCHER.** An Efficient Intracavity Laser Raman Spectrometer. *Applied Spectroscopy,* 1978, vol. 32 (3), 298-301
- **DAHMANI et al.** Frequency Stabilization of Semi-conductor lasers by resonant optical feedback. *Opt. Lett.,* 1987, vol. 12, 876-878
- **PETER BUCH et al.** Optically Self-Locked Semiconductor Laser with Servo Control for Feedback Phase and Laser Current. *IEEE Journal of Quantum Electronics,* 1991, vol. 27 (7), 1863-1868
- **M. HIPPLER ; C. MOHR ; K. A. KEEN ; E. D. MC-NAGHTEN.** Cavity-enhanced resonant photoacoustic spectroscopy with optical feedback cw diode lasers: A novel technique for ultratrace gas analysis and high-resolution spectroscopy. *The Journal of Chemical Physics,* 2010, vol. 133, 044308
- **M. HIPPLER.** Cavity-Enhanced Raman Spectroscopy of Natural Gas with Optical Feedback cw-Diode Lasers. *Anal. Chem.,* 2015, vol. 87, 7803-7809